# EUROPEAN PATENT APPLICATION

(11) **EP 4 759 319 A1**
(43) Date of publication of application: **17.06.2026**
(21) Application number: 24851948.0
(22) Date of filing: 09.08.2024
(51) Int. Cl.: A61K 35/19, A61P 19/00, A61P 21/00, C07K 14/62, C07K 14/79, C12N 5/077, C12N 5/078, C12N 5/0775, C12N 9/26, C12N 9/50, C12N 9/66, C12N 9/76

(54) **TREATMENT OF TENDON DISORDERS USING PLATELETS PRODUCED FROM ADIPOSE TISSUE-DERIVED MESENCHYMAL STEM CELL LINE**

(30) Priority: 10.08.2023 JP 2023131108
(71) Applicant: Keio University, Tokyo, 108-8345 (JP)
(72) Inventor: SATO, Kazuki, Tokyo 160-8582 (JP); TORII, Akiko, Tokyo 160-8582 (JP); YAMADA, Yuichi, Tokyo 160-8582 (JP); URUGA, Yukako, Tokyo 160-8582 (JP); MATSUBARA, Yumiko, Tokyo 160-8582 (JP)
(74) Representative: Script Intellectual Property LLP
(86) International application number: PCT/JP2024/028622
(87) International publication number: WO 2025/033531

(57) **Abstract**

An object of the present invention is to provide, for example, a therapeutic agent for tendinopathy using platelet produced from a mesenchymal stem cell line derived from an adipose tissue (ASCL), the therapeutic agent stably having an excellent therapeutic effect on the tendinopathy. The present invention includes, for example, a therapeutic agent for tendinopathy, the therapeutic agent comprising platelet as an active ingredient, the platelet having been produced by a production method comprising the following steps (A) to (C): (A) inducing one or more cells selected from a stromal vascular fraction of a vertebrate animal adipose tissue comprising a mesenchymal stem cell, an adipose progenitor cell, and a stromal cell to differentiate into a mature adipocyte; (B) inducing the mature adipocyte obtained in step (A) to dedifferentiate to obtain a mesenchymal stem cell line derived from the vertebrate animal adipose tissue; and (C) culturing the mesenchymal stem cell line derived from the adipose tissue obtained in step (B) in a medium for inducing differentiation into megakaryocytic cells, and collecting platelet from the cultures, the medium comprising an iron ion and an iron transporter.

## Description

### Technical Field

The present invention relates to, for example, a therapeutic agent for tendinopathy, the therapeutic agent comprising platelet produced from a mesenchymal stem cell line derived from an adipose tissue (adipose-derived mesenchymal stem cell line: ASCL) as an active ingredient.

### Background Art

Tendinopathy is a disorder based on chronic inflammation at muscle-tendon attachment sites for hand or finger movement, for example, and includes tendinitis, enthesitis, and the like. The tendinopathy appears more commonly in the joints of the extremities, such as the elbows (lateral humeral epicondylitis (so-called tennis elbow) and medial humeral epicondylitis (so-called golf elbow)), the heels (Achilles tendonitis), the shoulders (Rotator cuff tendonitis), and the knees (patellar tendinitis), and causes a sharp pain during action, for example, holding things, turning a doorknob, or walking, posing an obstacle not only to work and sporting activity but to daily performance. In general, the tendinopathy is caused by degeneration or microtear at a tendon attachment site ascribable to repetitive motions in work or sports, or aging. Its symptoms become refractory without being improved once developed, and many cases suffer from pains over years. Such a disorder is also called refractory enthesitis.

Previous epidemiological investigation on lateral humeral epicondylitis (so-called tennis elbow), which is representative tendinitis or enthesitis with a high incidence rate, has reported that the incidence rate is 1.0 to 1.3% for males and 1.1 to 4.0% for females with a peak age of onset from 30s to 50s. 10 to 20% of these cases are refractory and eventually require surgery. However, surgery for patients under the age of 65 has been reported to have poor performance because the patients have high postoperative activities. Middle-aged or older males, i.e., core workers in the society, are limited by their social activities, leading to reduced productivity and social loss. This can be predicted from results in which musculoskeletal diseases occupied a proportion as large as 38.1% of occupational diseases and, particularly, lateral humeral epicondylitis and finger tendinitis were most frequent. Tendon injury (tendinitis, enthesitis, etc.) typified by lateral humeral epicondylitis is also considered as a disease that cannot be overlooked from a medical economic standpoint.

Although a wide variety of treatment methods such as internal or external use of non-steroidal anti-inflammatory agents, local injection of steroids, physical therapy, ultrasound therapy, extracorporeal shock wave therapy, orthotic therapy, and surgical treatment are applied to treatment of tendinopathy, any definitive treatment method has not yet been established. Meanwhile, a report on the effectiveness of PRP (platelet-rich-plasma) therapy for tendinopathy such as tendinitis or enthesitis has received attention in recent years. For example, patent document 1 describes a freeze-dried polymer composition for forming an implant for tissue repair by mixing with PRP (platelet-rich plasma) or blood, and/or a composition for intra-articular injection for treatment.

Administered PRP is known to exert the therapeutic effects as mentioned above by releasing various cytokines. However, the amounts of the cytokines released by PRP differ largely among different donors for blood collection or among different PRP preparation methods even if the same donor is used, for example. Thus, PRP has large variation in quality. Particularly, platelet, because of its properties unique to the cells, is easily activated by physical stimulation during collection or preparation so that the cells aggregate, largely influencing the quality of PRP. Furthermore, a dose or an administration method varies among facilities, and there exists no constant protocol. Moreover, PRP is difficult to preserve while maintaining quality, and is administered immediately after collection without confirmation of the quality under the current circumstances.

Against this backdrop, it is difficult for PRP to conduct clinical research with a high evidence level, and "Humeral Lateral Epicondylitis Clinical Practice Guidelines 2019" states that the degree of recommendation remains on 2 (do is weakly recommended) and the evidence level is B (there is a moderate level of confidence in the estimate of a treatment's effect). In Class III regenerative medicine provision plans which are provided as private practice, PRP is administered to patients under the current circumstances while its effect has not been verified with high-level evidence. This situation occurs not only in Japan but in the USA, and almost all review papers on PRP state the quality of PRP as limitations.

Adipose tissue-derived mesenchymal stem cells which are cultured and proliferated from adipose tissues are known to participate in tissue regeneration by differentiation or to promote tissue regeneration by secretion of a growth factor. A treatment method of administering adipose tissue-derived mesenchymal stem cells (also called Adipose tissue-derived stem cells: ADSCs) by injection to a joint of a knee osteoarthritis patient has been clinically introduced as regenerative medicine around the world and is also performed in private practice in Japan. For example, patent document 2 states that adipose tissue-derived mesenchymal stem cells can be used in treatment of knee osteoarthritis. The knee osteoarthritis is a disease that causes joint pain, swelling, deformity, or the like by wear and tear on the cartilage of the knee joint, for example, due to the aging, overuse, or the like of the knee joint, and this disease is different from tendinopathy.

Meanwhile, the present inventors have previously developed a method for inducing differentiation into megakaryocytes and/or platelet by culturing adipose progenitor cells in a basal medium for mesenchymal cell culture containing an iron ion and an iron transporter (patent document 3), and a novel method for producing a mesenchymal stem cell line derived from an adipose tissue (ASCL) from mesenchymal stem cells derived from a vertebrate animal adipose tissue or the like (i.e., a novel cell line establishment method) (patent document 4). The present inventors have further developed a method for treating a wound by administering a platelet-like cell population produced from adipose tissue-derived mesenchymal stem cells (for example, a mesenchymal stem cell line derived from an adipose tissue) to the wound (patent document 5), etc. The present inventors are pursuing clinical application as to a technique of producing platelet from ASCL.

### Prior Art Documents

### Patent Documents

Patent Document 1: Japanese unexamined Patent Application Publication (Translation of PCT Application) No. 2017-506116
Patent Document 2: Japanese unexamined Patent Application Publication No. 2017-93431
Patent Document 3: International Publication No. WO 2014/208100
Patent Document 4: Domestic re-publication No. 2017/094260 of PCT international application
Patent Document 5: Japanese unexamined Patent Application Publication No. 2019-34917

### Summary of the invention

### Object to be Solved by the Invention

Current tendinopathy treatment using PRP generally employs patients themselves as PRP donors. The current treatment method had some problems. The first problem of the treatment method using PRP is that PRP has large variation in quality among different individuals (donors) for blood collection or among different PRP preparation methods. The second problem is that PRP is difficult to secure in a sufficient amount because PRP is obtained only in a small amount from blood. The third problem is that the treatment method using PRP requires an invasive operation of collecting blood from patients and also requires medical institutes to aseptically prepare PRP from the blood and immediately administer the PRP to the patients, before the PRP loses its therapeutic activity. This also disadvantageously poses various burdens both on the patients and on the medical institutes.

An object of the present invention is to provide, for example, a therapeutic agent for tendinopathy using platelet produced from a mesenchymal stem cell line derived from an adipose tissue (ASCL), the therapeutic agent stably having an excellent therapeutic effect on the tendinopathy.

### Means to Solve the Object

The present inventors conducted extensive studies to solve the above problems and found that, for example,
(a) in the case of using platelet produced from ASCL, a tendinopathy model rat has a significantly low IL-6 concentration and can significantly suppress inflammation, as compared with the case of using PRP (platelet-rich plasma), and
(b) platelet contained in PRP produces no TGF-β (one kind of anti-inflammatory cytokine) by stimulation with calcium, whereas the platelet produced from ASCL produced TGF-β (one kind of anti-inflammatory cytokine) by stimulation with calcium,
whereby the present invention was accomplished.

Even those skilled in the art have not expected that the platelet produced from ASCL has the marked effects as described above, as compared with platelet derived from blood, such as PRP.

More specifically, the present invention includes the following aspects:
(1) a therapeutic agent for tendinopathy, the therapeutic agent comprising platelet as an active ingredient, the platelet having been produced by a production method comprising the following steps (A) to (C):
   (A) inducing one or more cells selected from a stromal vascular fraction of a vertebrate animal adipose tissue comprising a mesenchymal stem cell, an adipose progenitor cell, and a stromal cell to differentiate into a mature adipocyte;
   (B) inducing the mature adipocyte obtained in step (A) to dedifferentiate to obtain a mesenchymal stem cell line derived from the vertebrate animal adipose tissue; and
   (C) culturing the mesenchymal stem cell line derived from the adipose tissue obtained in step (B)in a medium for inducing differentiation into megakaryocytic cells, and collecting platelet from the cultures, the medium comprising an iron ion and an iron transporter;
(2) the therapeutic agent according to the above (1), wherein the step of inducing one or more cells to differentiate into a mature adipocyte in step (A) is a step of culturing the one or more cells in a basal medium for mesenchymal cell culture, the basal medium comprising one or more adipose cell differentiation inducing substances selected from the group consisting of dexamethasone, isobutylmethylxanthine, insulin, and serum;
(3) the therapeutic agent according to the above (1) or (2), wherein inducing the mature adipocyte to dedifferentiate in step (B) is performing a ceiling culture of the mature adipocyte;
(4) the therapeutic agent according to any one of the above (1) to (3), wherein the one or more cells are a cell obtained by removing the mature adipocyte from a cell population obtained by treating the vertebrate animal adipose tissue with an enzyme capable of dispersing vertebrate animal adipose tissue cells;
(5) the therapeutic agent according to the above (4), wherein the enzyme capable of dispersing the vertebrate animal adipose tissue cells is one or more enzymes selected from the group consisting of collagenase, trypsin, caseinase, clostripain, trypsin-EDTA, dispase, thermolysin, pronase, hyaluronidase, pancreatin, elastase, and papain;
(6) the therapeutic agent according to any one of the above (1) to (5), wherein the iron transporter in the step (C) is transferrin;
(7) the therapeutic agent according to any one of the above (1) to (5), wherein the iron ion and the iron transporter in the step (C) are transferrin bound to iron;
(8) the therapeutic agent according to any one of the above (1) to (7), wherein the therapeutic agent is administered to the tendon of a vertebrate animal in need of treatment of the tendinopathy;
(9) the therapeutic agent according to any one of the above (1) to (8), wherein the tendinopathy is selected from the group consisting of tendinosis, tendinitis, and tenosynovitis;
(10) the therapeutic agent according to any one of the above (1) to (9), wherein the tendinopathy is selected from the group consisting of Achilles tendinopathy, patellar tendinopathy, lateral epicondylitis, medial epicondylitis, plantar fasciitis, and rotator cuff tendinopathy; and
(11) the therapeutic agent according to any one of the above (1) to (10), wherein the vertebrate animal is a human.

### Effect of the Invention

The present invention can provide, for example, a therapeutic agent for tendinopathy using platelet produced from a specific mesenchymal stem cell line derived from an adipose tissue (ASCL), the therapeutic agent stably having an excellent therapeutic effect on the tendinopathy. The platelet of the present invention has the advantages that, for example, the platelet can be cultured in a large amount and can also be cryopreserved because the platelet is prepared from ASCL.

### Brief Description of Drawings

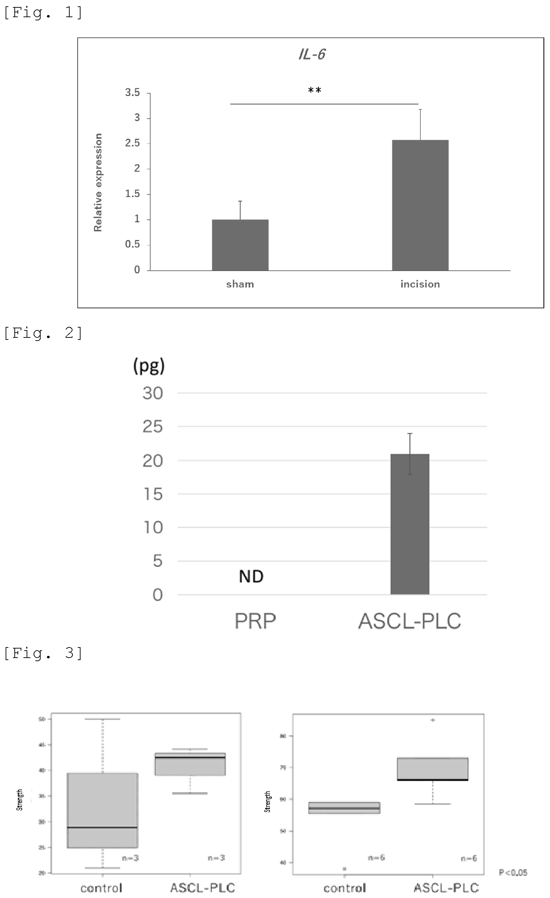

[Figure 1] Figure 1 is a diagram showing results of analyzing an expression level of IL-6 (one kind of inflammatory cytokine) by RT-PCR in the leg on the incision side and the leg on the sham side in a model rat with tendon injury. The numeric values on the ordinate represent relative expression levels when the expression level in the leg on the sham side is defined as 1.
   "**" in the diagram represents statistically significant difference from the control group (p < 0.01) (the same holds true for the description below).
[Figure 2] Figure 2 is a diagram showing results of stimulating 1 × 10⁸ cells of ASCL-PLC or PRP with 10 mM CaCl₂ by incubation for 15 minutes in PBS (phosphate buffered saline), and then measuring an amount (pg) of TGF-β extracellularly released into the PBS by platelet activated by stimulation.
[Figure 3] Figure 3 is a diagram showing results of conducting a maximum breaking load test on the Achilles tendon in an ASCL-PLC administration group (left legs) and a control group (PBS administration group) (right legs) of model rats with tendon degeneration. The left panel depicts results obtained after 2 weeks from the start of administration of ASCL-PLC or PBS, and the right panel depicts results obtained after 4 weeks from the start of administration of ASCL-PLC or PBS. The ordinates of both the panels depict maximum breaking loads (N (newton)) on the Achilles tendon.

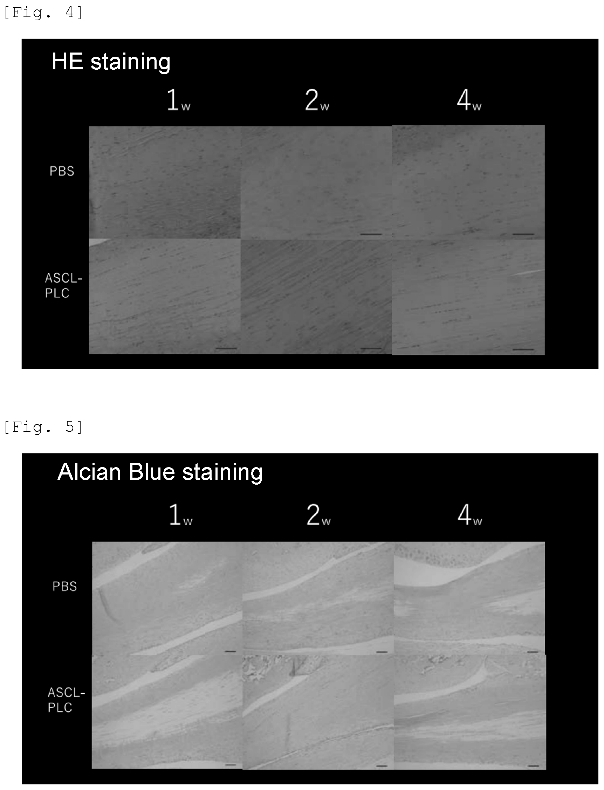

[Figure 4] Figure 4 is a diagram showing results of fixing each tissue section of the Achilles tendon in formalin for an ASCL-PLC administration group (left legs) and a control group (PBS administration group) (right legs) of model rats with tendon degeneration, followed by hematoxylin-eosin (HE) staining. The panels of the left column depict results obtained after 1 week from the start of administration of ASCL-PLC or PBS, the panels of the middle column depict results obtained after 2 weeks from the start of administration of ASCL-PLC or PBS, and the panels of the right column depict results obtained after 4 weeks from the start of administration of ASCL-PLC or PBS.
[Figure 5] Figure 5 is a diagram showing results of fixing each tissue section of the Achilles tendon in formalin for an ASCL-PLC administration group (left legs) and a control group (PBS administration group) (right legs) of model rats with tendon degeneration, followed by Alcian Blue staining. The panels of the left column depict results obtained after 1 week from the start of administration of ASCL-PLC or PBS, the panels of the middle column depict results obtained after 2 weeks from the start of administration of ASCL-PLC or PBS, and the panels of the right column depict results obtained after 4 weeks from the start of administration of ASCL-PLC or PBS.

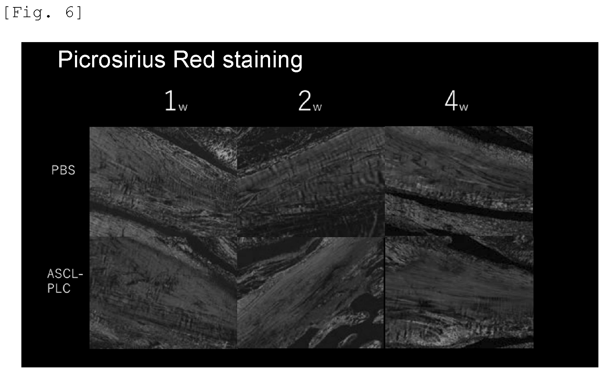

[Figure 6] Figure 6 is a diagram showing results of fixing each tissue section of the Achilles tendon in formalin for an ASCL-PLC administration group (left legs) and a control group (PBS administration group) (right legs) of model rats with tendon degeneration, followed by Picrosirius Red staining. The panels of the left column depict results obtained after 1 week from the start of administration of ASCL-PLC or PBS, the panels of the middle column depict results obtained after 2 weeks from the start of administration of ASCL-PLC or PBS, and the panels of the right column depict results obtained after 4 weeks from the start of administration of ASCL-PLC or PBS.

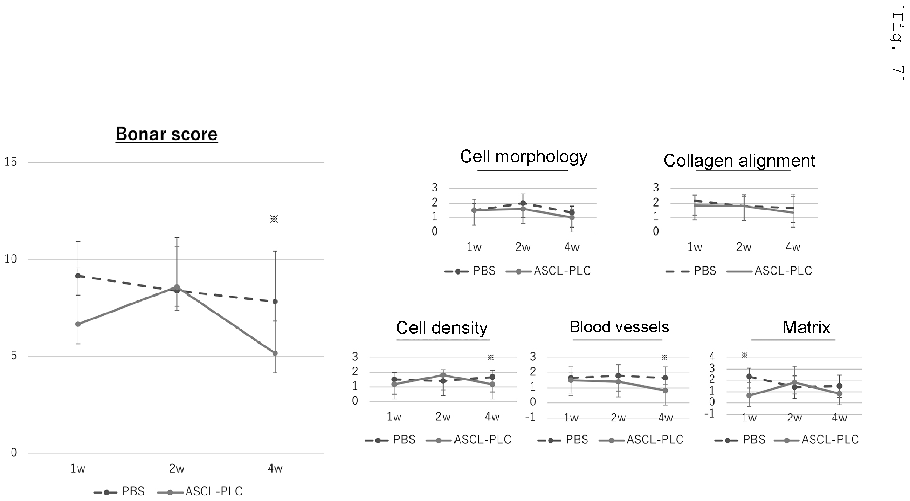

[Figure 7] Figure 7 is a diagram showing results of calculating Bonar scores from the results of the hematoxylin-eosin staining of Figure 5 and the Alcian Blue staining of Figure 6, and comparing the results about the control group (PBS administration group) and the ASCL-PLC administration group on a weekly basis after administration. The solid lines depict the results about the ASCL-PLC administration group, and the broken lines depict the results about the control group (PBS administration group). Results about five factors used in the calculation of the Bonar scores, i.e., "cell morphology", "collagen alignment", "cell density", "blood vessels", and "matrix", are also shown. "*" in the diagram represents statistically significant difference from the control group (p < 0.05) (the same holds true for the description below).

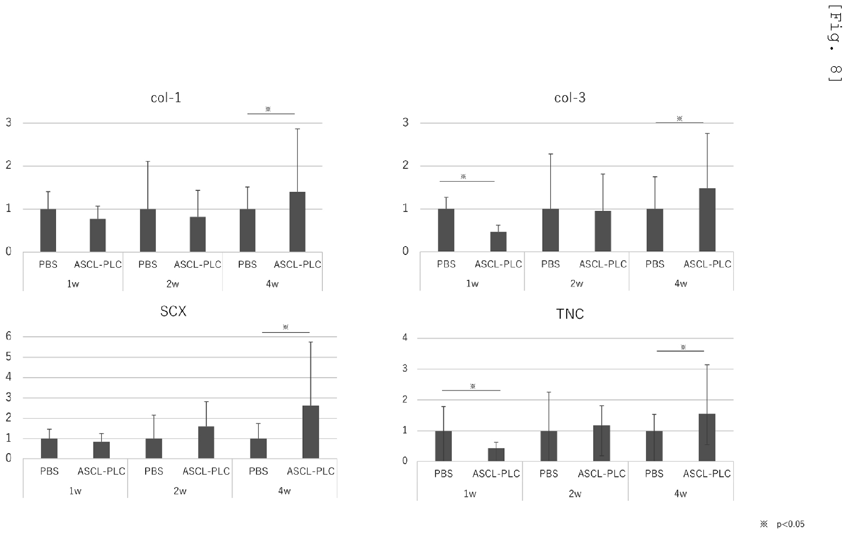

[Figure 8] Figure 8 is a diagram showing results of analyzing expression levels of tendon-related cytokines (i.e., Col-1, Col-3, SCX, and TNC) by RT-PCR in an ASCL-PLC administration group (left legs) and a control group (PBS administration group) (right legs) of model rats with tendon degeneration. The numeric values on the ordinates represent relative expression levels when the expression levels in the control group (PBS administration group) are defined as 1. 1w depicts results obtained after 1 week from the start of administration of ASCL-PLC or PBS, 2w depicts results obtained after 2 weeks from the start of administration of ASCL-PLC or PBS, and 4w depicts results obtained after 4 weeks from the start of administration of ASCL-PLC or PBS.

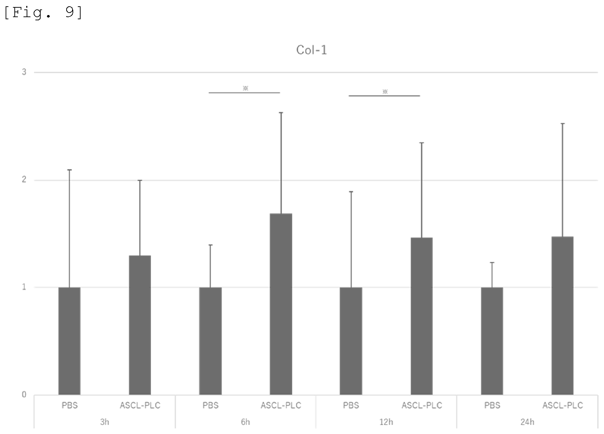

[Figure 9] Figure 9 is a diagram showing results of contacting tenoblasts with PBS (phosphate buffered saline) containing 1 × 10⁸ cells of ASCL-PLC, or PBS (control) for 3 hours, 6 hours, or 12 hours, followed by the measurement of an amount of Col-1 in the PBS. The numeric values on the ordinate represent relative expression levels when the expression level in the control group (PBS administration group) is defined as 1

### Mode of Carrying Out the Invention

The present invention includes an embodiment of
[1] a therapeutic agent for tendinopathy (hereinafter also referred to as "therapeutic agent of the present invention" in the present specification), the therapeutic agent comprising platelet (hereinafter also referred to as "platelet according to the present invention" in the present specification) as an active ingredient, the platelet having been produced by a production method (hereinafter also referred to as "production method according to the present invention" in the present specification) comprising the following steps (A) to (C):
   (A) inducing one or more cells selected from a stromal vascular fraction of a vertebrate animal adipose tissue comprising a mesenchymal stem cell, an adipose progenitor cell, and a stromal cell to differentiate into a mature adipocyte;
   (B) inducing the mature adipocyte obtained in step (A) to dedifferentiate to obtain a mesenchymal stem cell line derived from the vertebrate animal adipose tissue (hereinafter also referred to as "cell line according to the present invention" in the present specification) ; and
   (C) culturing the mesenchymal stem cell line derived from the adipose tissue obtained in step (B) in a medium for inducing differentiation into megakaryocytic cells, and collecting platelet from the cultures, the medium comprising an iron ion and an iron transporter.

The present invention also includes, as other embodiments, embodiments of:
[2] a method for treating tendinopathy, the method comprising the step of administering the platelet according to the present invention to a subject in need of treatment of the tendinopathy;
[3] the platelet according to the present invention for use in treatment of tendinopathy;
[4] use of the platelet according to the present invention for producing a therapeutic agent for tendinopathy;
[5] the platelet according to the present invention for use as a therapeutic agent for tendinopathy; etc.

### (Therapeutic agent of present invention)

The therapeutic agent of the present invention is not particularly limited as long as the therapeutic agent is a therapeutic agent for tendinopathy, the therapeutic agent comprising platelet as an active ingredient, the platelet having been produced by a production method comprising the following steps (A) to (C):
(A) inducing one or more cells selected from a stromal vascular fraction of a vertebrate animal adipose tissue comprising a mesenchymal stem cell, an adipose progenitor cell, and a stromal cell to differentiate into a mature adipocyte;
(B) inducing the mature adipocyte obtained in step (A) to dedifferentiate to obtain a mesenchymal stem cell line derived from the vertebrate animal adipose tissue; and
(C) culturing the mesenchymal stem cell line derived from the adipose tissue obtained in step (B) in a medium for inducing differentiation into megakaryocytic cells, and collecting platelet from the cultures, the medium comprising an iron ion and an iron transporter.

The mature adipocyte obtained by the differentiation induction in step (A) is a mature adipocyte more easily dedifferentiated (hereinafter also referred to as "easy-to-dedifferentiate mature adipocyte" in the present Description) than a mature adipocyte which have been present in a vertebrate animal adipose tissue, and thus the dedifferentiation induction in step (B) presumably enables the produce (establishment) of a mesenchymal stem cell line derived from a vertebrate animal adipose tissue (ASCL) more simply, in a shorter period of time, and more efficiently. The present inventors have already confirmed that ASCL produced from the easy-to-dedifferentiate mature adipocyte (i.e., ASCL produced by a method comprising the steps (A) and (B) according to the present invention) differs as a cell from a mesenchymal stem cell line derived from an adipose tissue produced by directly inducing a mature adipocyte obtained from a vertebrate animal adipose tissue to dedifferentiate. The "ASCL" as used herein refers to a mesenchymal stem cell line derived from an adipose tissue produced by the method comprising the steps (A) and (B) according to the present invention.

The above production method according to the present invention comprising the steps (A) to (C) can be an ex vivo or in vitro production method.

### (Platelet according to present invention)

The platelet according to the present invention is not particularly limited as long as it is platelet produced by the above production method comprising the steps (A) to (C) (the production method according to the present invention). The production method according to the present invention is not particularly limited as long as it is a production method comprising the above steps (A) to (C).

### (Step A)

The above step (A) is not particularly limited as long as it is a step of inducing one or more cells selected from a stromal vascular fraction of a vertebrate animal adipose tissue comprising a mesenchymal stem cell, an adipose progenitor cell, and a stromal cell (also referred to as "mesenchymal stem cells" in the present Description) to differentiate into a mature adipocyte. The step of differentiation induction is an ex vivo or in vitro step of differentiation induction.

The species from which the adipose tissue is derived is not particularly limited as long as a species is a vertebrate animal, and examples include a mammal, a bird, a reptile, an amphibian, and a fish, of which a mammal such as a human, a mouse, a rat, a guinea pig, a rabbit, a cat, a dog, a horse, a cow, a monkey, a sheep, a goat, and a pig being preferable, of which a human being particularly preferable. For a mesenchymal stem cell line derived from a vertebrate animal adipose tissue produced by the method for producing a cell line of the present invention, the adipose tissue of the target vertebrate animal may be used in the method for producing a cell line of the present invention in light of minimizing a rejection reaction or the like.

The "adipose tissue" as used herein is not particularly limited as long as a tissue comprises fats and examples include a subcutaneous adipose tissue, an adipose tissue in the bone marrow, and a visceral adipose tissue, with a subcutaneous adipose tissue being preferable in light of comparatively low invasiveness to a vertebrate animal supplying the adipose tissue and being comparatively easily collectable.

The "stromal vascular fraction" as used herein means the cells other than mature adipocytes among the cells of a vertebrate animal adipose tissue. A stromal vascular fraction typically comprises cells such as a mesenchymal stem cell, an adipose progenitor cell, a stromal cell, a vascular endothelial cell, a cell related to blood, a smooth muscle cell, and a fibroblast. The "stromal vascular fraction" can be obtained by removing mature adipocytes from a cell population obtained by treating a vertebrate animal adipose tissue with an enzyme capable of dispersing vertebrate animal adipose tissue cells.

The above "one or more cells selected from a stromal vascular fraction of a vertebrate animal adipose tissue comprising a mesenchymal stem cell, an adipose progenitor cell, and a stromal cell" are not limited as long as one or more cells are selected from a stromal vascular fraction of a vertebrate animal adipose tissue comprising a mesenchymal stem cell, a preadipocyte or an adipose progenitor cell, and a stromal cell, but in light of more efficiently producing a mesenchymal stem cell line derived from a vertebrate animal adipose tissue, the cell population, rather than comprising only an adipose progenitor cell, preferably comprises at least an adipose progenitor cell and a mesenchymal stem cell and/or a stromal cell, with the cell population comprising at least an adipose progenitor cell, a mesenchymal stem cell, and a stromal cell being more preferable, with the cell population of a stromal vascular fraction being further preferable in light of easy preparation.

Further, examples of the preferable embodiment of the above "one or more cells selected from a stromal vascular fraction of a vertebrate animal adipose tissue comprising a mesenchymal stem cell, an adipose progenitor cell, and a stromal cell" include one or more cells selected from a stromal vascular fraction comprising a mesenchymal stem cell, an adipose progenitor cell, and a stromal cell obtained by dispersing cells of the vertebrate animal adipose tissue, of which a cell population obtained by removing mature adipocytes from a cell population obtained by treating the vertebrate animal adipose tissue with an enzyme capable of dispersing vertebrate animal adipose tissue cells (cell population A) being preferable. A cell population obtained by further removing vascular endothelial cells and/or cells related to blood from the cell population A may also be used. The above cell population (cell population A) obtained by removing mature adipocytes from a cell population obtained by treating a vertebrate animal adipose tissue with an enzyme capable of dispersing the vertebrate animal adipose tissue cells is a cell population of stromal vascular fractions, which typically comprises cells such as a mesenchymal stem cell, an adipose progenitor cell, a stromal cell, a vascular endothelial cell, a cell related to blood, a smooth muscle cell and a fibroblast of a vertebrate animal adipose tissue.

Examples of the above "treating a vertebrate animal adipose tissue with an enzyme capable of dispersing vertebrate animal adipose tissue cells" include a method in which a vertebrate animal adipose tissue is immersed in a solution comprising such an enzyme and incubated, for example, for about 30 minutes to 3 hours.

The above "enzyme capable of dispersing vertebrate animal adipose tissue cells" is not particularly limited as long as it can disperse cells of a vertebrate animal adipose tissue when allowed to act on the vertebrate animal adipose tissue, and examples include one or more enzymes selected from the group consisting of collagenase, trypsin, caseinase, clostripain, trypsin-EDTA, dispase, thermolysin, pronase, hyaluronidase, pancreatin, elastase, and papain, of which at least one or more enzymes selected from the group consisting of collagenase, trypsin, caseinase, and clostripain being preferable, and commercial collagenase (type I) and collagenase (type II) being more preferable, with collagenase (type II) being further preferable. Further, the above "enzyme capable of dispersing vertebrate animal adipose tissue cells" preferably comprises at least collagenase.

The above "removing mature adipocytes from a cell population obtained by treating a vertebrate animal adipose tissue with an enzyme capable of dispersing vertebrate animal adipose tissue cells" is not particularly limited as long as a method can remove mature adipocytes from such a cell population, but examples preferably include a method of recovering a cell population (cell pellet) which is precipitated by centrifugation of a suspension comprising the above cell population. Mature adipocytes comprise a large amount of fats, thus have a light specific gravity and float in the upper part of supernatant when centrifuged, hence the recovery of a cell pellet precipitated by the centrifugation enables the removal of mature adipocytes. Further, the method for removing vascular endothelial cells, smooth muscle cells, and fibroblasts from the cell population obtained by treating a vertebrate animal adipose tissue with an enzyme capable of dispersing vertebrate animal adipose tissue cells is not particularly limited as long as a method can remove these cells from such a cell population and examples include a method for removing vascular endothelial cells from the cell population when CD31 known as a surface marker of the vascular endothelial cell selects negative cells (or CD31 removes positive cells), and examples of the method for removing cells related to blood include a method for removing cells related to blood from the cell population by selecting CD45- (a surface marker of hematopoietic cells other than red blood cell and platelet) negative and Ter119- (a surface marker of red blood cell and progenitor cell thereof) negative cells (or CD45-positive and Ter119-positive cells are removed). Additionally, when 7-amino-actinomycin D (7-AAD), which is not a surface marker, being negative is used as an indicator, it is preferable because dead cells comprised in a vertebrate animal adipose tissue can be excluded. 7-AAD intercalates with a DNA chain of a dead cell and produces red fluorescence at a 488-nm excitation light.

The above precipitated cell pellet (cell population A) is cells of a stromal vascular fraction, which typically comprises a mesenchymal stem cell, an adipose progenitor cell, a stromal cell (a stroma cell), a vascular endothelial cell, a smooth muscle cell, and a fibroblast, however, those of which differentiable into a mature adipocyte is a mesenchymal stem cell, an adipose progenitor cell, and a stromal cell. For this reason, a further step may or may not be included for removing any one or more, or all kinds, of the cells other than these 3 kinds from the above precipitated cell pellet before the differentiation induction into a mature adipocyte is carried out, but it is preferable not to include such a step in light of convenience of operation. A vascular endothelial cell, a smooth muscle cell, and a fibroblast do not differentiate into a mature adipocyte even when induced with mesenchymal stem cells to differentiate into a mature adipocyte or do not interfere in the differentiation of mesenchymal stem cells into mature adipocytes.

In the above step (A), examples of the method for inducing one or more cells selected from a stromal vascular fraction of a vertebrate animal adipose tissue comprising a mesenchymal stem cell, an adipose progenitor cell, and a stromal cell to differentiate into a mature adipocyte preferably include a method for culturing the one or more cells selected from a stromal vascular fraction of a vertebrate animal adipose tissue comprising a mesenchymal stem cell, an adipose progenitor cell, and a stromal cell in a basal medium for mesenchymal cell culture comprising adipose cell differentiation inducing substances. The method for culturing mesenchymal stem cells in a basal medium for mesenchymal cell culture comprising adipose cell differentiation inducing substances is not particularly limited as long as a method can induce mesenchymal cells to differentiate into mature adipocytes by such a culture, and the same method as the typical method for inducing an adipose progenitor cell to differentiate into a mature adipocyte, that is, a method for culturing a starting cell in a basal medium for mesenchymal cell culture comprising adipose cell differentiation inducing substances can be used.

In the above step (A), examples of the conditions for culturing mesenchymal stem cells in a basal medium for mesenchymal cell culture comprising adipose cell differentiation inducing substances include a method of adhesion culture in a culture vessel coated with the extracellular matrix, and examples of the culture temperature include typically a range from 12 to 45°C, preferably a range from 15 to 37°C, and examples of the culture period include, in light of the balance between producing a mesenchymal stem cell line derived from a vertebrate animal adipose tissue more efficiently and producing in a shorter period of time, a range from 5 to 16 days, preferably a range from 7 to 14 days, more preferably a range from 8 to 12 days, further preferably a range from 9 to 11, more preferably for 10 days. In the culture, mesenchymal stem cells may or may not be subcultured. Further, examples of the above extracellular matrix include at least one or more components selected from collagen, fibronectin, proteoglycan, and laminin, and BD Matrigel (registered trademark) (manufactured by BD Biosciences) comprising these components can also be used.

The above adipose cell differentiation inducing substances are not particularly limited as long as a substance has an action to differentiate a cell inducible to differentiate into a mature adipocyte or has an assisting action on the action, and examples include one or more substances selected from the group consisting of dexamethasone, isobutylmethylxanthine, insulin, and serum, of which, in light of obtaining a good differentiation inducing efficiency into a mature adipocyte, "combination of serum and dexamethasone", "combinations of adipose cell differentiation inducing substances comprising at least serum and dexamethasone", "combination of serum and isobutylmethylxanthine", and "combinations of adipose cell differentiation inducing substances comprising at least serum and isobutylmethylxanthine" being preferable, of which "combination of serum, dexamethasone, and insulin", "combinations of adipose cell differentiation inducing substances comprising at least serum, dexamethasone, and insulin", "combination of serum, isobutylmethylxanthine, and insulin", "combinations of adipose cell differentiation inducing substances comprising at least serum, isobutylmethylxanthine and insulin", "combination of serum, dexamethasone, and isobutylmethylxanthine", and "combinations of adipose cell differentiation inducing substances comprising at least serum, dexamethasone, and isobutylmethylxanthine" being more preferable, of which "combination of serum, dexamethasone, isobutylmethylxanthine, and insulin", "combinations of adipose cell differentiation inducing substances comprising at least serum, dexamethasone, isobutylmethylxanthine, and insulin" being further preferable. The adipose cell differentiation inducing substances and the basal medium for mesenchymal cell culture comprising such a substance may be commercial products, or a medium prepared by adding an adipose cell differentiation inducing substance to the basal medium for mesenchymal cell culture may be used as such a medium. Examples of the commercial medium comprising an adipose cell differentiation inducing substance preferably include Adipocyte Differentiation Medium (manufactured by Cell Applications, Inc.). Examples of the substance having an assisting action on the action to differentiate into a mature adipocyte other than the above listed adipose cell differentiation inducing substances include Rosiglitazone, Pioglitazone, and Indomethacin.

The concentration of the above adipose cell differentiation inducing substances in the medium is not particularly limited as long as a concentration can induce mesenchymal stem cells into mature adipocytes but examples include, in terms of dexamethasone concentration, typically a range from 0.1 to 10 µM, preferably a range from 0.5 to 2.5 µM, in terms of isobutylmethylxanthine concentration, a range from 10 to 1000 µM, preferably a range from 250 to 750 µM, in terms of insulin concentration, a range from 0.1 to 10 µM, preferably a range from 0.5 to 2.5 µM, and in terms of serum concentration, a range from 1 to 20 wt%, preferably a range from 5 to 15 wt%, more preferably 7 to 13 wt%.

The "basal medium for mesenchymal cell culture" in the present Description is not particularly limited as long as medium can culture at least 1 kind of the mesenchymal cells therein and proliferate the mesenchymal cell, but in light of easy preparation and preventing lot-to-lot variation, a chemically synthesized medium is preferable, and the medium preferably comprises one or more saccharide(s), one or more inorganic salt(s), one or more amino acid(s), and one or more vitamin(s), and one or more other components as needed.

Examples of the above saccharide specifically include a monosaccharide such as glucose, mannose, fructose and galactose, and a disaccharide such as sucrose, maltose, and lactose, of which glucose being particularly preferable, and one or more of these saccharides can be added in combination.

Examples of the above inorganic salt specifically include one or more inorganic salt(s) such as calcium chloride, calcium nitrate, a copper sulfate pentahydrate, an iron(III) nitrate nonahydrate, an iron (II) sulfate heptahydrate, a magnesium chloride hexahydrate, magnesium sulfate, potassium chloride, sodium chloride, sodium bicarbonate, disodium hydrogen phosphate, a disodium hydrogenphosphate dihydrate, sodium dihydrogen phosphate, a sodium dihydrogen phosphate monohydrate, a sodium dihydrogen phosphate dihydrate, a sodium selenite pentahydrate, and a zinc sulfate heptahydrate.

Examples of the above amino acids specifically include one or more amino acid(s) selected from alanine, arginine, asparagine, aspartic acid, cystine, cysteine, glutamine, glycine, histidine, glutamic acid, hydroxyproline, isoleucine, leucine, lysine, methionine, phenylalanine, proline, serine, threonine, tryptophan, tyrosine, and valine, and preferably include an L-form amino acid and a derivative thereof, and products derived therefrom such as a salt thereof and a hydrate thereof. Examples of the above arginine include products derived from arginine such as an L-arginine hydrochloride and an L-arginine monohydrochloride, examples of the above aspartic acid include products derived from aspartic acid such as an L-sodium aspartate monohydrate, an L-aspartic acid monohydrate, potassium L-aspartate, and magnesium L-aspartate, examples of the above cysteine include products derived from cysteine such as L-cysteine dihydrochloride and an L-cysteine hydrochloride monohydrate, and products derived from lysine such as L-lysine hydrochloride, examples of the above glutamic acid include products derived from glutamine such as monosodium L-glutamate, examples of the above asparagine include products derived from asparagine such as an L-asparagine monohydrate, examples of the above tyrosine include products derived from tyrosine such as an L-tyrosine disodium dihydrate, examples of the above histidine include products derived from histidine such as histidine hydrochloride and a histidine hydrochloride monohydrate, and examples of the above lysine include products derived from lysine such as L-lysine hydrochloride.

Examples of the above vitamins specifically include one or more vitamin(s) selected from biotin, choline, folic acid, inositol, niacin, pantothenic acid, pyridoxine, riboflavin, thiamine, vitamin B12, paraaminobenzoic acid (PABA), and ascorbic acid, and a derivative each thereof, and products derived therefrom such as a salt thereof and a hydrate thereof. Examples of the above choline include products derived from choline such as choline chloride, examples of the niacin include products derived from niacin such as nicotinic acid, nicotinamide, and nicotinic alcohol, examples of the pantothenic acid include products derived from pantothenic acid such as calcium pantothenate, sodium pantothenate, and panthenol, examples of the pyridoxine include products derived from pyridoxine such as pyridoxine hydrochloride, pyridoxal hydrochloride, pyridoxal phosphate, and pyridoxamine, examples of the thiamine include products derived from thiamine such as thiamine hydrochloride, thiamine nitrate, bisthiamine nitrate, a thiamine dicetyl sulfate ester salt, fursultiamine hydrochloride, octothiamine, and benfotiamine, examples of the ascorbic acid include products derived from ascorbic acid such as ascorbic acid 2-phosphate, ascorbic acid magnesium phosphate, sodium ascorbate sulfate, aminopropyl ascorbyl phosphate, and sodium ascorbate phosphate.

Examples of the above other components include a buffer such as HEPES, an antibiotic such as penicillin and streptomycin, pyruvic acid and a derivative thereof, and products derived therefrom such as a salt thereof and a hydrate thereof, and phenol red, examples of products derived from the above antibiotics include penicillin G sodium and streptomycin sulfate, or preferably a penicillin-streptomycin solution, and examples of products derived from the pyruvic acid preferably include sodium pyruvate.

Specific examples of the above basal medium for mesenchymal cell culture include a known commercial chemically synthesized medium such as Dulbecco's modified Eagle's medium (DMEM), Iscove's Modified Dulbecco's Medium (IMDM), RPMI1640 medium, minimum essential medium (MEM), basal medium of Eagle (BME), and F12 medium, a medium of 2 or more of these medium mixed in a suitable ratio such as DMEM/F12 (medium of DMEM and F12 medium mixed in 1:1), with medium to which one or more substances selected from the group consisting of antibiotics such as penicillin and streptomycin; and additional amino acids (preferably non-essential amino acids); are added being preferable, and medium wherein an antibiotic (preferably penicillin G sodium, streptomycin sulfate or a penicillin-streptomycin solution) is further added to DMEM, IMDM or RPMI1640 medium being particularly more preferable, of which medium wherein an antibiotic (preferably penicillin G sodium, streptomycin sulfate, or a penicillin-streptomycin solution) is further added to DMEM being particularly preferable.

Examples of the particularly preferable basal medium for mesenchymal cell culture in the present invention include medium wherein 100 U/mL (final concentration) of a penicillin-streptomycin solution is added to DMEM having the composition to be described later (hereinafter referred to as "particularly preferable basal medium in the present invention"), and medium comprising each component in a concentration of the proportion ranging independently from 70% to 130% (preferably ranging from 80 to 120 wt%) to the concentration of each component in the particularly preferable basal medium in the present invention.

### (Composition of DMEM)

200 mg/L of anhydrous calcium chloride, 0.1 mg/L of Fe(NO₃)₃•9H₂O, 200 mg/L of potassium chloride, 97.67 mg/L of anhydrous magnesium sulfate, 6400 mg/L of sodium chloride, 3700 mg/L of sodium bicarbonate, 125 mg/L of sodium dihydrogen phosphate monohydrate, 4500 mg/L of D-glucose, 15 mg/L of phenol red, 110 mg/L of sodium pyruvate, 84 mg/L of L-arginine hydrochloride, 63 mg/L of L-cysteine dihydrochloride, 584 mg/L of L-glutamine, 30 mg/L of glycine, 42 mg/L of L-histidine hydrochloride monohydrate, 105 mg/L of L-isoleucine, 105 mg/L of L-leucine, 146 mg/L of L-lysine hydrochloride, 30 mg/L of L-methionine, 66 mg/L of L-phenylalanine, 42 mg/L of L-serine, 95 mg/L of L-threonine, 16 mg/L of L-tryptophan, 104 mg/L of L-tyrosine disodium dihydrate, 94 mg/L of L-valine, 4 mg/L of D-calcium pantothenate, 4 mg/L of choline chloride, 4 mg/L of folic acid, 7.2 mg/L of i-inositol, 4 mg/L of nicotinamide, 4 mg/L of pyridoxine hydrochloride, 0.4 mg/L of rivoflavin, 4 mg/L of thiamine hydrochloride.

Mature adipocytes obtained in the above step (A) (that is, the mature adipocyte population comprising mature adipocytes) are easy-to-dedifferentiate mature adipocytes, which are comparatively easily dedifferentiated when induced to dedifferentiate (that is, easy-to-dedifferentiate mature adipocyte population comprising easy-to-dedifferentiate mature adipocytes). The "easy-to-dedifferentiate mature adipocyte population" in the present Description means a mature adipocyte population having a proportion of the cell line obtained which is 1.5 or more times more than that of the mature adipocyte population collected from a vertebrate animal adipose tissue as described in the conventional method (Japanese Patent No. 5055611), and includes mature adipocyte populations having preferably 2 or more times, more preferably 4 or more times, further preferably 6 or more times, more preferably 10 or more times, and further preferably 15 or more times proportions. The above "proportion of the cell line obtained" indicates the proportion of cell line obtained from a specific amount of a mature adipocyte population, and the proportion preferably includes, for example, a proportion (rate) of "a weight of a cell line to be obtained" to "a weight of mature adipocytes to be used for dedifferentiation induction."

### (Step B)

The above step (B) is not particularly limited as long as a step can induce the mature adipocytes (easy-to-dedifferentiate mature adipocytes) obtained in step (A) to dedifferentiate to obtain a mesenchymal stem cell line derived from a vertebrate animal adipose tissue. The step is an ex vivo or in vitro step.

Mature adipocytes used in step (B) is the mature adipocytes obtained by the differentiation induction in step (A). Such mature adipocytes can be obtained by, for example, centrifuging the culture suspension of step (A) and collecting the cells which float in the upper part of the supernatant. This is because mature adipocytes comprise a large amount of fats, thus have a light specific gravity and float in the upper part of supernatant when centrifuged.

In the above step (B), the method of inducing the mature adipocytes (easy-to-dedifferentiate mature adipocytes) obtained in step (A) to dedifferentiate to obtain a mesenchymal stem cell line derived from a vertebrate animal adipose tissue is not particularly limited as long as a method can induce the mature adipocytes to dedifferentiate to obtain a mesenchymal stem cell line derived from a vertebrate animal adipose tissue, but examples preferably include a method of so-called ceiling culture of the mature adipocytes. The ceiling culture is a method for culturing cells by allowing the cells to adhere or float (preferably allowed to adhere) to the inner upper surface (ceiling surface) of a culture vessel (preferably a culture flask) filled up with medium, and this method for culturing cells utilizes the property of mature adipocytes which comprise a large amount of fats, thus have a light specific gravity and float in the medium.

Examples of the medium when carrying out the dedifferentiation inducing culture of a mature adipocyte include basal medium for mesenchymal cell culture comprising the extracellular matrix, and examples of the extracellular matrix include one or more components selected from collagen, fibronectin, proteoglycan, laminin, and serum (FBS), and BD Matrigel (registered trademark) (manufactured by BD Biosciences) comprising such a component can also be used. Serum such as FBS in medium when carrying out the dedifferentiation inducing culture of a mature adipocyte may be used only as an adhesion factor for allowing a mature adipocyte to adhere to the ceiling surface of a culture vessel or may not be used only as the adhesion factor for that purpose. The medium when carrying out the dedifferentiation inducing culture of a mature adipocyte may not comprise serum such as FBS, but in light of producing a mesenchymal stem cell line derived from a vertebrate animal adipose tissue more efficiently, it is preferable to comprise serum such as FBS with the extracellular matrix other than serum or without the extracellular matrix other than serum. The serum concentration, in the case where the medium comprises serum such as FBS, is not particularly limited as long as a mesenchymal stem cell line derived from a vertebrate animal adipose tissue is obtained but examples include a range from 3 to 30 wt%, preferably include a range from 7 to 25 wt%, more preferably include a range from 7 to 13 wt%.

In the above step (B), when the conditions, other than the ceiling culture conditions, for culturing a mature adipocyte in the basal medium for mesenchymal cell culture comprising the extracellular matrix are described, examples of the culture temperature typically include a range from 12 to 45°C, preferably a range from 15 to 37°C, and examples of the culture period include a range from 2 to 28 days, preferably a range from 4 to 21 days, more preferably a range from 5 to 14 days, further preferably a range from 6 to 10 days, more preferably for 7 days, in light of balancing the production of a mesenchymal stem cell line derived from a vertebrate animal adipose tissue between more efficiently and in a shorter period of time. In the culture, a mature adipocyte may or may not be subcultured.

A mesenchymal stem cell line derived from a vertebrate animal adipose tissue may or may not be isolated from the medium after the ceiling culture in the above step (B), but it is preferable to isolate. When the ceiling culture is continued, mature adipocytes gradually decrease while the established mesenchymal stem cell line derived from an adipose tissue actively proliferates and a cell population comprising a large amount of mesenchymal stem cell lines derived from an adipose tissue can be obtained. For example, when the ceiling culture is continued for about 14 days, a cell population containing an extremely large amount of mesenchymal stem cell lines derived from an adipose tissue can be obtained.

The ceiling culture in the above step (B) include, for the sake of convenience, the continuation of culture in which after the mature adipocyte (easy-to-dedifferentiate mature adipocyte) obtained in step (A) is adhered to the ceiling surface of a culture vessel, the culture vessel is arranged in such a way that the adhesion surface turns to the bottom side thereof, however, the culture may be continued while the mature adipocyte (easy-to-dedifferentiate mature adipocyte) obtained in step (A) are adhered to the ceiling surface of a culture vessel to obtain a mesenchymal stem cell line derived from an adipose tissue without carrying out the culture in which the culture vessel is arranged in such a way that the adhesion surface turns to the bottom side of the medium.

The mesenchymal stem cell line derived from an adipose tissue (ASCL) produced by a method comprising the steps (A) and (B) according to the present invention is not particularly limited as long as it is a mesenchymal stem cell line derived from a vertebrate animal adipose tissue produced by the method comprising the steps (A) and (B) according to the present invention. ASCL owned by Heartseed Inc. is particularly preferable.

The ASCL according to the present invention does not spontaneously differentiate when cultured in the typical basal medium for mesenchymal cell culture which does not have a differentiation inducing action, is suitable for long-term subculture, and maintains proliferation potency and differentiation potency into mesodermal cells (one or more cells selected from the group consisting of a megakaryocyte/platelet, an osteoblast, a cartilage, and an adipocyte) even after long-term subculture. For example, The ASCL according to the present invention produced from a human subcutaneous adipose tissue has been observed to maintain proliferation potency even in the 20th generation and have a doubling time of 23 hours.

The ASCL according to the present invention preferably expresses one or more (preferably 3 or more, more preferably 5 or more, further preferably 7 or more, more preferably 8 or 9, most preferably 9) surface markers selected from the following surface marker group of mesenchymal cells, and does not express one or more (preferably 3 or more, more preferably 5 or more, further preferably 7 or more, more preferably 8 or 9, most preferably 9) surface markers selected from the following surface marker group of blood cells.
Surface marker group of mesenchymal cells: CD13, CD29, CD44, CD71, CD73, CD90, CD105, CD166, HLA-ABC;
Surface marker group of blood cells: CD11b, CD14, CD19, CD34, CD41, CD42b, CD45, CD56, HLA-DR;

International Society for Cellular Therapy sets conditions to define the mesenchymal stem cell as (a) to be an adherent cell, (b) to be capable of differentiating into bones, cartilages, and fats, and (c) to express surface markers of mesenchymal cells and not express surface markers of blood cells. Of the ASCLs according to the present invention, the cell lines of a preferable embodiment meet the conditions (a), (b), and (c).

The ASCL according to the present invention can be proliferated by culture in a mesenchymal stem cell proliferation medium. A commercially available product can be used as the mesenchymal stem cell proliferation medium.

### (Step C)

The above step (C) is not particularly limited as long as it is the step of culturing the mesenchymal stem cell line derived from the adipose tissue obtained in step (B) in a medium for inducing differentiation into megakaryocytic cells, and collecting platelet from the cultures, the medium comprising an iron ion and an iron transporter. The step of differentiation induction is an ex vivo or in vitro step of differentiation induction. A detailed mechanism of action is unknown under which platelet is produced from the ASCL according to the present invention when the ASCL is cultured in a medium for inducing differentiation into megakaryocytic cells, the medium comprising an iron ion and an iron transporter. The iron ion, or the iron ion and the iron transporter taken up into the ASCL promote thrombopoietin (TPO) secretion from the cells through some mechanism, and this is considered partly responsible for promoting the induction of differentiation of the ASCL into platelet.

The "medium for inducing differentiation into megakaryocytic cells, the medium comprising an iron ion and an iron transporter" in the step (C) (hereinafter also referred to as "medium of the step (C)") is a medium further containing an iron ion and an iron transporter in a basal medium for mesenchymal cell culture. The iron ion and the iron transporter further contained therein cause an action of inducing ASCL to differentiate into platelet.

The above iron ion may be any of an iron ion(II) and an iron ion (III) , and iron ion (III) is preferable. Examples of the method for allowing the iron ion to be contained in the medium of the step (C) include a method of adding one or more iron salts selected from the group consisting of inorganic salts and organic salts of iron to the medium for inducing differentiation into megakaryocytic cells. The iron salt may be an organic salt or an inorganic salt. Examples of the inorganic salt include iron(II) chloride, iron(III) chloride, iron(II) oxide, iron(III) oxide, iron(II) nitrate, iron(III) nitrate, iron(II) sulfate, iron(III) sulfate, iron(II) ammonium sulfate, iron(III) ammonium sulfate, iron(II) pyrophosphate, iron(III) pyrophosphate, iron(II) sulfide, iron(III) sulfide, iron(II) hydroxide, and iron(III) hydroxide. Examples of the above organic salt include iron(II) acetate, iron(III) acetate, hydroxydiacetoxy iron(III), iron(II) citrate, iron(III) citrate, iron(III) sodium citrate, iron(III) ammonium citrate, iron(II) benzoate, iron(III) benzoate, iron(II) carbonate, iron(III) carbonate, iron(II) formate, iron(III) formate, iron(II) oxalate, iron(III) oxalate, iron(II) fumarate, iron(III) fumarate, iron(II) succinate, iron(III) succinate, iron(II) gluconate, iron(III) gluconate, iron(II) lactate, iron(III) lactate, iron(II) maleate, iron(III) maleate, iron(III) sodium diethylenetriaminepentaacetate, iron(III) ammonium diethylenetriaminepentaacetate, iron(III) sodium ethylenediaminetetraacetate, iron(III) ammonium ethylenediaminetetraacetate, iron(III) sodium dicarboxymethylglutamate, and iron(III) ammonium dicarboxymethylglutamate. One of these iron salts may be used, or two or more thereof may be used in combination. Commercially available products can be used as these iron salts.

The above iron transporter binds to the iron ion contained in the medium of the step (C) and provides the ability of ASCL to take up the iron ion from the medium. The iron transporter bound to the iron ion, if used, also functions as a supply source of iron. The iron transporter may be called apo form when not bound to iron, called holo form when bound to iron, and called sidero form when bound to iron in an amount at an intermediate level between the apo form and the holo form. Examples of the above iron transporter include a protein that binds to iron so as to be taken up into cells (Japanese unexamined Patent Application Publication No. 08-029429, Japanese unexamined Patent Application Publication (Translation of PCT Application) No. 2005-517042, Japanese unexamined Patent Application Publication (Translation of PCT Application) No. 2004-505932, Japanese unexamined Patent Application Publication (Translation of PCT Application) No. 2007-508026, etc.) and preferably include, as the apo form or iron transporters corresponding thereto, apo-transferrin (apo-serotransferrin), apo-lactoferrin, apo-ovotransferrin, apo-melanotransferrin, apo-ferritin, and protoporphyrin IX. Among them, apo-transferrin is preferable. The species from which the iron transporter is derived is preferably the same species as that from which mesenchymal cells used together are derived.

A complex formed by the binding between the iron ion and the iron transporter (iron ion-iron transporter complex) can be preferably used as the iron ion and the iron transporter according to the present invention. Examples of the iron ion-iron transporter complex include holo-transferrin in which apo-transferrin is bound to the iron ion (transferrin bound to iron), holo-lactoferrin in which apo-lactoferrin is bound to the iron ion (lactoferrin bound to iron), holo-ovotransferrin in which apo-ovotransferrin is bound to the iron ion (ovotransferrin bound to iron), holo-melanotransferrin in which apo-melanotransferrin is bound to the iron ion (melanotransferrin bound to iron), holo-ferritin in which apo-ferritin is bound to the iron ion (ferritin bound to iron), and hem in which protoporphyrin IX is bound to iron. Among them, transferrin bound to iron is particularly preferable. Commercially available products can be used as the holo form of the above iron transporter bound to the iron ion, the apo form of the iron transporter not bound to the iron ion, and the sidero form of the iron transporter bound to iron in an amount at an intermediate level between the apo form and the holo form.

The binding mode between the iron ion and the iron transporter in the above iron ion-iron transporter complex is not particularly limited and may be a noncovalent bond such as a coordinate bond, an ionic bond, a hydrogen bond, a metal bond, or Van der Waals' force, or may be a covalent bond. A coordinate bond is preferable because the degree of binding is moderate and the bond is suitable for transporting the iron ion into ASCL. In the iron ion-iron transporter complex, the iron is not in the state of an ion. However, a complex that is capable of releasing the iron ion when taken up into ASCL is also included in the iron ion-iron transporter complex according to the present invention for the sake of convenience.

The concentration of the iron ion in the medium of the step (C) is not particularly limited as long as the medium for use in the present invention having such an iron concentration can culture ASCL therein and produce the platelet of the present invention. Examples thereof include a range from 1 pg/mL to 10 pg/mL, preferably a range from 10 pg to 1 µg/mL, more preferably a range from 150 pg/mL to 300 pg/mL, and further preferably a range from 150 pg/mL to 250 pg/mL.

The content of the iron transporter in the medium of the step (C) is not particularly limited as long as the medium for use in the present invention having such a content of the iron transporter can culture ASCL therein and produce the platelet of the present invention. Examples thereof include a range from 10 fM (1 × 10⁻¹⁵ M) to 100 nM, preferably a range from 100 fM to 10 nM, more preferably a range from 1 pM to 2.8 pM, and further preferably a range from 1 pM to 2.5 pM.

In the case of using transferrin bound to iron in the medium of the step (C), its concentration of addition is not particularly limited and may abide by the above numeric range of the iron ion concentration. Examples thereof include 25 µg/mL to less than 400 µg/mL, preferably 50 pg/mL to less than 200 µg/mL. 1 mg of the transferrin bound to iron reportedly contains about 1.3 µg of the bound iron ion.

The medium of the step (C) is not particularly limited as long as the medium can culture ASCL therein and produce the platelet of the present invention, when supplemented with the iron ion and the iron transporter according to the present invention, but in light of easy preparation and preventing lot-to-lot variation, a chemically synthesized medium is preferable, and the medium preferably comprises one or more saccharide(s), one or more inorganic salt(s), one or more amino acid(s), and one or more vitamin(s), and one or more other components.

As mentioned above, the medium (medium for inducing differentiation into megakaryocytic cells) of the step (C) is a medium containing an iron ion and an iron transporter in a basal medium for mesenchymal cell culture that can culture mesenchymal cells. Preferable specific examples of the basal medium for mesenchymal cell culture for use in the medium of the step (C) include a known commercial chemically synthesized medium such as commercially available Iscove's Modified Dulbecco's Medium (IMDM), RPMI 1640 medium, Dulbecco's modified Eagle's medium (DMEM), minimum essential medium (MEM), basal medium of Eagle (BME), and F12 medium, a medium of 2 or more of these medium mixed in a suitable ratio such as DMEM/F12 (medium of DMEM and F12 medium mixed in 1:1), with medium to which a nucleic acid such as a nucleotide, antibiotics such as penicillin and streptomycin, and L-glutamine are added being preferable, and medium wherein an antibiotic (preferably penicillin G sodium, streptomycin sulfate or a penicillin-streptomycin solution) and L-glutamine are further added to IMDM or RPMI 1640 medium being particularly more preferable, of which medium wherein an antibiotic (preferably penicillin G sodium, streptomycin sulfate, or a penicillin-streptomycin solution) and L-glutamine are further added to IMDM being particularly preferable.

Examples of the particularly preferable basal medium for mesenchymal cell culture for use in the medium of the step (C) include medium wherein 2 mM (final concentration) L-glutamine and 100 U/mL (final concentration) of a penicillin-streptomycin solution are added to IMDM having the composition to be described later (hereinafter referred to as "particularly preferable basal medium for use in the medium of the step (C)"), and medium comprising each component in a concentration of the proportion ranging independently from 70 to 130 wt% (preferably ranging from 80 to 120 wt%) to the concentration of each component in the particularly preferable basal medium for use in the medium of the step (C).

### (Composition of IMDM)

0.4 mM glycine, 0.281 mM L-alanine, 0.398 mM L-arginine hydrochloride, 0.167 mM L-asparagine, 0.226 mM L-aspartic acid, 0.381 mM L-cystine dihydrochloride, 0.51 mM L-glutamic acid, 4 mM L-glutamine, 0.2 mM L-histidine hydrochloride monohydrate, 0.802 mM L-isoleucine, 0.802 mM L-leucine, 0.798 mM L-lysine hydrochloride, 0.201 mM L-methionine, 0.4 mM L-phenylalanine, 0.348 mM L-proline, 0.4 mM L-serine, 0.798 mM L-threonine, 0.0784 mM L-tryptophan, 0.462 mM L-tyrosine disodium dihydrate, 0.803 mM L-valine, 0.0000533 mM biotin, 0.0286 mM choline chloride, 0.00839 mM calcium D-pantothenate, 0.00907 mM folic acid, 0.0328 mM nicotinamide, 0.0196 mM pyridoxal hydrochloride, 0.00106 mM riboflavin, 0.119 mM thiamine hydrochloride, 0.0000096 mM vitamin B12, 0.04 mM i-inositol, 1.49 mM anhydrous calcium chloride, 0.84 mM anhydrous magnesium sulfate, 4.4 mM potassium chloride, 0.000752 mM potassium nitrate, 36 mM sodium bicarbonate, 77.59 mM sodium chloride, 0.906 mM sodium dihydrogen phosphate monohydrate, 0.0000658 mM sodium selenite pentahydrate, 25 mM D-glucose, 25.03 mM HEPES, 0.0399 mM phenol red, and 1 mM sodium pyruvate.

Particularly preferable examples of the medium for use in the step (C) include a medium containing an iron ion and an iron transporter in the particularly preferable basal medium for use in the medium of the step (C), with a medium containing transferrin bound to iron as a single active ingredient in the particularly preferable basal medium for use in the medium of the step (C) being more preferable.

Although the medium of the step (C) may be supplemented with TPO, bovine serum albumin (BSA), LDL cholesterol, insulin, 2-β-mercaptoethanol, or the like, the absence of such addition is more meaningful in such a way that the platelet-like cell population of the present invention can be produced at lower cost. More specifically, MKLI medium, which has heretofore been used as a medium for inducing differentiation into megakaryocytic cells, is supplemented with IMDM medium as well as TPO, BSA, LDL cholesterol, insulin, and 2-β-mercaptoethanol (hereinafter, these components are also collectively referred to as "five components") (Matsubara Y, Murata M, Ikeda Y. Culture of megakaryocytes and platelets from subcutaneous adipose tissue and a preadipocyte cell line. Methods Mol Biol. 2012; 788: 249-258), whereas the medium of the step (C), without use of these five components, can produce the platelet-like cell population of the present invention at lower cost. The present inventors experimentally confirmed that none of BSA, LDL cholesterol, insulin, and 2-β-mercaptoethanol are necessary for inducing ASCL to differentiate into megakaryocytes or platelet. Among the media of the step (C) (i.e., "media for inducing differentiation into megakaryocytic cells, the media comprising an iron ion and an iron transporter"), a medium that comprises none of bovine serum albumin, LDL cholesterol, deoxyribonucleotide triphosphate, and 2-mercaptoethanol and comprises human serum albumin, transferrin bound to iron, insulin, and monothioglycerol is referred to as "modified medium for inducing differentiation into megakaryocytic cells" in the present specification. When ASCL can take up the iron ion in the medium into the cells in the absence of the iron transporter in the medium, the medium for use in the present invention may be interpreted as "medium for inducing differentiation into megakaryocytic cells, the medium comprising an iron ion", not "medium for inducing differentiation into megakaryocytic cells, the medium comprising an iron ion and an iron transporter".

A particularly preferable embodiment of the medium of the step (C) is a medium in which human serum albumin, transferrin bound to iron, insulin, and monothioglycerol are added to the particularly preferable basal medium for use in the medium of the step (C), and further examples of the medium include a medium comprising none of bovine serum albumin, LDL cholesterol, deoxyribonucleotide triphosphate, and 2-mercaptoethanol, more specifically, modified MKLI medium having the following composition, and medium comprising each component in a concentration of the proportion ranging independently from 70% to 130% (preferably ranging from 80 to 120 wt%) to the concentration of each component in the medium.

The modified MKLI medium can be prepared by adding 2 mM L-glutamine (manufactured by Life Technologies Japan Ltd.), 100 U/mL of a penicillin-streptomycin solution (manufactured by Life Technologies Japan Ltd.), 0.5% human serum albumin (manufactured by Sigma-Aldrich), 200 µg/mL iron saturated transferrin (manufactured by Sigma-Aldrich), 10 µg/mL insulin (manufactured by Sigma-Aldrich), 20 pM monothioglycerol (manufactured by FUJIFILM Wako Pure Chemical Corp.) to IMDM medium (Iscove's modified Dulbecco's medium, manufactured by Life Technologies Japan Ltd.). The modified MKLI medium having this composition or the medium comprising each component in a concentration of the proportion ranging independently from 70% to 130% (preferably ranging from 80 to 120 wt%) to the concentration of each component in the medium is preferably included in the "modified medium for inducing differentiation into megakaryocytic cells". The modified MKLI medium differs from conventional MKLI medium in that human serum albumin is used instead of BSA; no LDL cholesterol is used; no dNTP is used; and monothioglycerol is used instead of mercaptoethanol.

In the above step (C), the conditions for culturing the mesenchymal stem cell line derived from the adipose tissue obtained in step (B) in the medium for inducing differentiation into megakaryocytic cells, the medium comprising an iron ion and an iron transporter are not particularly limited as long as the medium of the step (C) can culture ASCL therein and produce the platelet according to the present invention. Examples of the culture temperature include typically a range from 12 to 45°C, preferably a range from 15 to 37°C, and examples of the culture period include typically a range from 4 to 25 days, preferably a range from 5 to 17 days. Further examples thereof include a range from 8 to 17 days.

The production method according to the present invention preferably further comprises the step of increasing the number of cells by maintenance culture of the cell line according to the present invention, before culture of the cell line according to the present invention in the medium of the step (C). This is because the maintenance culture step, if present, can increase the number of cells of the cell line according to the present invention that can be used in culture in the medium of the step (C), as described later, and can drastically elevate the yield of the platelet according to the present invention based on the initially provided number of cells of the cell line according to the present invention. The medium for use in the maintenance culture is not particularly limited as long as the medium can proliferate the cell line according to the present invention. Examples thereof include the basal medium for mesenchymal cell culture (containing neither an iron ion nor an iron transporter) mentioned above. In the case of performing maintenance culture, a basal medium for mesenchymal cell culture comprising serum or a serum component is preferably used. In the maintenance culture step, it is preferable to appropriately perform subculture or medium replacement.

Examples of the method for collecting the platelet according to the present invention from the cultures in the step (C) include, but are not particularly limited to, a method of fractionating a culture supernatant containing the platelet according to the present invention from the cultures, and a method of separating the platelet according to the present invention depending on a molecular size using a filter or the like.

The method for producing the platelet according to the present invention may use the presence or absence of a specific cell surface marker or a specific combination of cell surface markers as an indicator and further comprise, when or after the platelet according to the present invention is collected from the cultures, the step of (a) selecting platelet having a specific cell surface marker profile (i.e., platelet expressing the specific cell surface marker or the specific combination of cell surface markers) from the platelet according to the present invention, or (b) improving or reducing the proportion of platelet having a specific cell surface marker profile in the platelet according to the present invention, but in light of convenience, etc., preferably comprises no such step. The proportion of the platelet expressing the specific cell surface marker or the specific combination of cell surface markers in the platelet that has undergone the selection step or the improvement or reduction step includes the proportions of cells positive to specific cell surface markers listed in the present specifications or all combinations of the proportions.

Preferred examples of the method for (a) selecting platelet having a specific cell surface marker profile from the platelet according to the present invention, or the method for (b) improving or reducing the proportion of platelet having a specific cell surface marker profile in the platelet according to the present invention include, but are not particularly limited to, a method of using an antibody (preferably a labeled antibody, and more preferably a fluorescently labeled antibody) against each of the above cell surface markers, and selecting platelet having a specific cell surface marker profile by using the presence or absence of specific binding of each antibody as an indicator, in light of more conveniently and rapidly selecting platelet having the target cell surface marker profile, or in light of improving or reducing the proportion of platelet having the target cell surface marker profile.

The above "selecting by using the presence or absence of specific binding of antibody as an indicator" means that for a positive cell surface marker in the profile, platelet that exhibits specific binding of the antibody against the marker is selected, and for a negative cell surface marker in the profile, platelet that does not exhibit specific binding of the antibody against the marker is selected. Examples of the method for selecting platelet having a specific cell surface marker profile by using the presence or absence of specific binding of the antibody as an indicator include, but are not particularly limited to, a method using a cell sorter, magnetic beads, or a column for cell adsorption, with a method using a cell sorter being preferable because of being more convenient and rapid. The method using a cell sorter is based on flow cytometry and well known to those skilled in the art. A specific method is described in instruction manuals of cell sorters as well as Japanese unexamined Patent Application Publication (Translation of PCT Application) No. 2009-513161, for example. The method using magnetic beads is well known as a magnetic separation method or the like to those skilled in the art, and specific examples of the method include a method of contacting magnetic beads carrying a specific antibody with cells, and then recovering the magnetic beads with a magnet so that platelet that specifically binds to the specific antibody is separated. The method using a column for cell adsorption is well known to those skilled in the art, and specific examples of the method include a method of contacting platelet with a column for cell adsorption carrying a specific antibody so that platelet other than the target platelet is adsorbed to the column.

A particularly preferable embodiment of the method for producing the platelet according to the present invention preferably has any one or both of the following features in light of convenience or obtaining a better therapeutic effect on tendinopathy:
after obtainment of a mesenchymal stem cell line derived from an adipose tissue in step (B) and before culture of the cell line in a medium for inducing differentiation into megakaryocytic cells, the medium comprising an iron ion and an iron transporter in the step (C), cell selection by using the presence or absence of expression of a specific cell surface marker as an indicator is not performed in the cell line; and
the method for collecting platelet from the cultures in the step (C) does not comprise cell selection by using the presence or absence of expression of a specific cell surface marker as an indicator.

The platelet according to the present invention co-expresses one or more platelet surface markers and one or more surface markers of mesenchymal cells.

In the present invention, "platelet population that co-expresses one or more platelet surface markers and one or more surface markers of mesenchymal cells" means a platelet population comprising platelet expressing one or more platelet surface markers (platelet positive to one or more platelet surface markers) and platelet expressing one or more surface markers of mesenchymal cells (platelet positive to one or more surface markers of mesenchymal cells), and may be a platelet population comprising platelet positive to one or more platelet surface markers and platelet positive to one or more surface markers of mesenchymal cells only as separate platelets, but is preferably a platelet population at least partially comprising platelet positive to one or more platelet surface markers and positive to one or more surface markers of mesenchymal cells.

In the present invention, the platelet population comprising the "platelet expressing one or more platelet surface markers (platelet positive to one or more platelet surface markers)" is not particularly limited as long as the platelet population comprises platelet expressing at least CD29. A platelet population comprising platelet further expressing one or more (preferably three) platelet surface markers selected from the group consisting of CD49b, CD42b, and CD41 is preferable. In the present invention, the "platelet population comprising the platelet expressing two or more platelet surface markers" means a platelet population comprising platelet expressing arbitrary one platelet surface marker (platelet positive to arbitrary one platelet surface marker) of the two or more platelet surface markers, and platelet expressing arbitrary one or more of the other platelet surface marker(s) (platelet positive to arbitrary one or more of the other platelet surface marker(s), and may be a platelet population comprising platelet positive to the above arbitrary one platelet surface marker and platelet positive to the above arbitrary one or more of the other platelet surface marker(s) only as separate platelets, but is preferably a platelet population at least partially comprising platelet positive to the above arbitrary one platelet surface marker and positive to the above arbitrary one or more of the other platelet surface marker(s).

Examples of the platelet population comprising platelet expressing the platelet surface marker CD29 specifically include a platelet population in which the proportion of CD29-positive platelet is preferably 60% or more, more preferably 70 to 90%, and further preferably 75 to 85%. Examples of the platelet population comprising platelet further expressing, in addition to CD29, one or more (preferably three) platelet surface markers selected from the group consisting of CD49b, CD42b, and CD41 specifically include, preferably, a platelet population in which the proportion of platelet positive to one or more (preferably three) platelet surface markers selected from the group consisting of CD49b, CD42b, and CD41 satisfies the following numeric range:
the proportion of CD49b-positive platelet is preferably 30% or more (more preferably 30 to 85%, and further preferably 30 to 70%);
the proportion of CD42b-positive platelet is preferably 5% or more (more preferably 6 to 80%, further preferably 7 to 60%, and more preferably 8 to 40%); and
the proportion of CD41-positive platelet is preferably 20% or more (more preferably 20 to 85%, and further preferably 30 to 70%).

In the present invention, the platelet population comprising the "platelet expressing one or more surface markers of mesenchymal cells (platelet positive to one or more surface markers of mesenchymal cells)" is not particularly limited as long as the platelet population comprises platelet expressing at least CD90. A platelet population comprising platelet further expressing one or more (preferably three or more, more preferably five or more, further preferably seven or more, and more preferably eight) surface markers of mesenchymal cells selected from the group consisting of CD13, CD26, CD44, CD73, CD77, CD81, CD95, and CD164 is preferable. In the present invention, the "platelet population comprising the platelet expressing two or more surface markers of mesenchymal cells" means a platelet population comprising platelet expressing arbitrary one surface marker of mesenchymal cells (platelet positive to arbitrary one surface marker of mesenchymal cells) of the two or more surface markers of mesenchymal cells, and platelet expressing arbitrary one or more of the other surface marker(s) of mesenchymal cells (platelet positive to arbitrary one or more of the other surface marker(s) of mesenchymal cells), and may be a platelet population comprising platelet positive to the above arbitrary one surface marker of mesenchymal cells and platelet positive to the above arbitrary one or more of the other surface marker(s) of mesenchymal cells only as separate platelets, but is preferably a platelet population at least partially comprising platelet positive to the above arbitrary one surface marker of mesenchymal cells and positive to the above arbitrary one or more of the other surface marker(s) of mesenchymal cells.

Examples of the platelet population comprising platelet expressing the surface marker CD90 of mesenchymal cells specifically include a platelet population in which the proportion of CD90-positive platelet is preferably 30% or more, more preferably 30 to 90%, and further preferably 35 to 70%. A platelet population is preferable in which the proportion of platelet further positive to, in addition to CD90, one or more (preferably three or more, more preferably five or more, further preferably seven or more, and more preferably eight) surface markers of mesenchymal cells selected from the group consisting of CD13, CD26, CD44, CD73, CD77, CD81, CD95, and CD164 satisfies the following numeric range:
the proportion of CD13-positive platelet is 30% or more (preferably 30 to 80%, and more preferably 35 to 65%);
the proportion of CD26-positive platelet is 15% or more (preferably 15 to 60%, and more preferably 20 to 45%);
the proportion of CD44-positive platelet is 30% or more (preferably 30 to 80%, and more preferably 35 to 65%);
the proportion of CD73-positive platelet is 40% or more (preferably 40 to 95%, and more preferably 45 to 80%);
the proportion of CD95-positive platelet is 20% or more (preferably 20 to 70%, and more preferably 25 to 55%); and
the proportion of CD164-positive platelet is 15% or more (preferably 15 to 55%, and more preferably 20 to 40%).

As mentioned above, the platelet population of the present invention is preferably a platelet population at least partially comprising platelet positive to one or more platelet surface markers and positive to one or more surface markers of mesenchymal cells and, specifically, the platelet population preferably satisfies the following proportion of positive platelet:
the proportion of CD42b-positive and CD90-positive platelet is 3% or more (preferably 3 to 80%, more preferably 5 to 40%, and further preferably 5 to 20%).

A preferable embodiment of the platelet population of the present invention is a platelet population in which the expression of one or more specific platelet surface markers (hereinafter also referred to as "low expressed platelet surface markers") is decreased as compared with the expression in a typical platelet population. The "platelet population in which the expression of one or more low expressed platelet surface markers is decreased as compared with the expression in a typical platelet population" means a platelet population in which the proportion of platelet positive to one or more low expressed platelet surface markers is lower than that in a typical platelet population. The "low expressed platelet surface markers" are one or more (preferably three or more, and more preferably five or more) markers selected from the group consisting of CD9, CD36, CD41/61, CD61, and CD147. The CD41/61 means a complex of CD41 and CD61, and the CD41/61-positive platelet means platelet expressing the complex of CD41 and CD61.

Specific examples of the platelet population in which the expression of one or more low expressed platelet surface markers is decreased as compared with the expression in a typical platelet population preferably include a platelet population in which the proportion of platelet positive to one or more (preferably three or more, and more preferably five or more) low expressed platelet surface markers selected from the group consisting of CD9, CD36, CD41/61, CD61, and CD147 satisfies the following numeric range:
the proportion of CD9-positive platelet is 30% or less (preferably 0 to 20%, and more preferably 0.5 to 150);
the proportion of CD36-positive platelet is 40% or less (preferably 0 to 30%, and more preferably 0.5 to 20%);
the proportion of CD41/61-positive platelet is 60% or less (preferably 0 to 40%, and more preferably 0.5 to 25%);
the proportion of CD61-positive platelet is 30% or less (preferably 0 to 10%, and more preferably 0.1 to 8%); and
the proportion of CD147-positive platelet is 50% or less (preferably 0 to 40%, and more preferably 1 to 30%).

A preferable embodiment of the platelet population of the present invention is a platelet population comprising platelet positive to one or more platelet surface markers and platelet positive to one or more surface markers of mesenchymal cells, and further comprising platelet positive to one or two surface markers of activated platelet, i.e., CD107a and/or CD107b. The platelet positive to surface markers of activated platelet may be platelet different from the platelet positive to one or more platelet surface markers or the platelet positive to one or more surface markers of mesenchymal cells. The platelet population of the present invention is preferably a platelet population at least partially comprising platelet positive to surface markers of activated platelet and positive to one or more platelet surface markers, or platelet positive to surface markers of activated platelet and positive to one or more surface markers of mesenchymal cells.

Specific examples of the platelet population comprising the platelet positive to one or two surface markers of activated platelet, i.e., CD107a and/or CD107b preferably include a platelet population in which the proportion of platelet positive to one or two surface markers of activated platelet, i.e., CD107a and/or CD107b, satisfies the following numeric range:
the proportion of CD107a-positive platelet is 15% or more (more preferably 25 to 80%, and further preferably 45 to 70%); and
the proportion of CD107b-positive platelet is 10% or more (more preferably 15 to 70%, and further preferably 20 to 55%) .

The platelet population of the present invention may be a platelet population in which (a) the presence or absence of a specific cell surface marker or a specific combination of cell surface markers is used as an indicator, and platelet having a specific cell surface marker profile (i.e., platelet expressing the specific cell surface marker or the specific combination of cell surface markers) is selected, or a platelet population in which (b) the proportion of platelet having a specific cell surface marker profile is improved or reduced, or may not be such a platelet population. The proportion of the platelet expressing the specific cell surface marker or the specific combination of cell surface markers in the platelet population includes the proportions of cells positive to specific cell surface markers listed in the present specifications or all combinations of the proportions.

A preferable embodiment of the platelet population of the present invention is a platelet population in which the amount of TGF-β (transforming growth factor β) produced measured by the following measurement method is 0.1 pg or more, preferably 1 pg or more, more preferably 2.5 pg or more, further preferably 5 pg or more, more preferably 7.5 pg or more, further preferably 10 pg or more, and more preferably 15 pg or more per 1 × 10⁸ cells of platelet.

### (Method for measuring amount of TGF-β produced)

Phosphate buffered saline containing 1 × 10⁸ cells/100 µL of platelet is stimulated with 10 mM CaCl₂ by incubation for 15 minutes, followed by the measurement of the amount of TGF-β in the phosphate buffered saline.

The composition of the PBS solution for use in the above suspension preferably includes 8 g/L NaCl, 0.2 g/L KCl, 1.44 g/L Na₂HPO₄, and 0.24 g/L KH₂PO₄ (pH 7.4).

The platelet of the present invention may not be platelet stimulated with calcium, but in light of obtaining a higher therapeutic effect on tendinopathy, is preferably platelet stimulated with calcium. Examples of the method for calcium stimulation include, but are not particularly limited to, a method of incubating the platelet of the present invention in a solution having a calcium concentration of, for example, 0.01 to 100 mM, preferably 0.5 to 50 mM, and more preferably 5 to 20 mM. Examples of the incubation time include, but are not particularly limited to, 0.1 minutes to 3 hours, preferably 10 to 20 minutes.

Examples of the compound containing calcium for use in calcium stimulation include, but are not particularly limited to, a calcium salt such as calcium chloride.

The timing when calcium stimulation is performed is not particularly limited, but is before administration of the platelet of the present invention, for example, within 5 hours before, within 3 hours before, within 1 hour before, or within 30 minutes before the timing of administration.

### (Therapeutic agent for tendinopathy of present invention)

The therapeutic agent of the present invention is not particularly limited as long as it contains the platelet according to the present invention as an active ingredient. The therapeutic agent of the present invention is capable of being prepared, stored, and administered with reference to findings about conventionally known cell preparations, or the like and in the same manner as therein. The therapeutic agent of the present invention usually has an injectable form. The therapeutic agent of the present invention can be administered into an affected tendon site (for example, Achilles tendon) in the target vertebrate animal (preferably, human patient).

The therapeutic agent of the present invention can be prepared into a preparation in a form suitable for administration to a subject, for example, by mixing the platelet according to the present invention with a pharmaceutically acceptable carrier. Examples of the carrier include physiological saline, and injectable distilled water rendered isotonic by the addition of glucose or other aids (for example, D-sorbitol, D-mannitol, and sodium chloride). The therapeutic agent of the present invention may be further supplemented with a buffer (for example, a phosphate buffer solution and a sodium acetate buffer solution), a soothing agent (e.g., benzalkonium chloride and procaine hydrochloride), a stabilizer (e.g., human serum albumin and polyethylene glycol), a preservative, an antioxidant, or the like.

The therapeutic agent of the present invention can also be used as a composition for tendinopathy treatment.

The therapeutic agent of the present invention may optionally further comprise, in addition to the platelet according to the present invention, various additive components generally used and other drugs effective for treatment of the tendinopathy.

The therapeutic target of the tendinopathy according to the present invention is not particularly limited as long as it is a vertebrate animal. Examples thereof include a mammal, a bird, a reptile, an amphibian, and a fish, of which a mammal such as a human, a mouse, a rat, a guinea pig, a rabbit, a cat, a dog, a horse, a cow, a monkey, a sheep, a goat, and a pig being preferable, of which a human being particularly preferable.

In the present specification, the "tendinopathy" is not particularly limited as long as it is a disorder in the tendon. Examples thereof include tendinosis, tendinitis, and tenosynovitis. More specifically, the tendinopathy is preferably tendinopathy selected from the group consisting of Achilles tendinopathy, patellar tendinopathy, lateral epicondylitis, medial epicondylitis, plantar fasciitis, and rotator cuff tendinopathy.

In the present specification, the treatment of tendinopathy includes not only improvement in symptoms of the tendinopathy but suppression or delay of worsening of symptoms of the tendinopathy, for example.

The dose per administration of the therapeutic agent of the present invention is not particularly limited as long as a therapeutic effect on tendinopathy is obtained. Those skilled in the art can appropriately set the dose in consideration of the severity of tendinopathy in a patient, the extent of the tendinopathy, etc. For example, 1 × 10⁵ to 1 × 10¹¹ cells of the platelet of the present invention can be used per administration. The number of doses of the therapeutic agent of the present invention is not particularly limited as long as a therapeutic effect on tendinopathy is obtained. Since even one dose produces an excellent therapeutic effect, only one dose may be administered, or two or more doses may be administered. Examples of the two or more doses include 1 to 7, 1 to 5, 1 to 3, and 1 to 2 doses. In the case of administering the therapeutic agent of the present invention twice or more, the dosing interval from previous administration can be appropriately determined according to articular symptoms of the target vertebrate animal. Examples thereof include an interval within the range of 3 weeks to 6 weeks and an interval within the range of 6 months to 1 year.

Hereinafter, the present invention will be described in detail with reference to Examples. However, the present invention is not limited by these examples.

### Examples

### 1. [Production of ASCL]

A mesenchymal stem cell line derived from an adipocyte (ASCL; adipose-derived mesenchymal stem cell line) was prepared in accordance with the method described in Examples of International Publication No. WO 2017/094260. Adipose-derived stem cells (ADSCs; adipose tissue-derived stem cells) were obtained from Cell Applications, Inc.

### 2. [Production of platelet of present invention]

The above ASCL obtained was cultured in modified MKLI medium for 14 days so that the ASCL differentiated into platelet. The platelet was collected from the medium to prepare the platelet of the present invention (hereinafter referred to as "ASCL-PLC").

### 3. [Preparation of PRP]

PRP for use in a control group was prepared. Specifically, peripheral blood was collected and centrifuged at 200 G for 10 minutes and subsequently at 1500 G for 10 minutes to obtain PRP.

### 4. [Preparation of model rat with tendon injury]

In order to confirm mainly the anti-inflammatory effect of the platelet according to the present invention, tendon injury models were adopted which were able to reliably induce inflammation above a given level. Ten-week-old Wistar rats (male) were used. The right leg of each rat was used as the sham side on which although Achilles tendon sheath incision was performed, excision was not made near an Achilles tendon attachment site (negative control). On the other hand, the left leg was used as the incision side on which Achilles tendon sheath incision was performed and, in addition, excision was made near an Achilles tendon attachment site.

### 5. [Analysis of expression level of inflammatory cytokine in tendon injury model]

An anti-inflammatory effect was verified by analyzing expression levels of inflammatory cytokines by RT-PCR using the tendon injury models. Three days after preparation of the model rats with tendon injury, each model rat with tendon injury was sacrificed, and the expression levels of the inflammatory cytokines (i.e., IL-6 and IL-1b) in the legs on the incision side and the sham side were analyzed by RT-PCR. Total RNA was extracted from the Achilles tendon using TRI reagent (TR118, Molecular Research Center, Inc, Cincinnati, OH) and Rneasy Mini Kit (74106, QIAGEN, Hamburg, Germany). cDNA was synthesized by reverse transcription using Advantage RT-for-PCR Kit (Takara Bio Inc., Otsu, Shiga, Japan). Real-time PCR (intercalator method) was carried out in DICE thermal cycler (manufactured by Takara Bio Inc.) using SYBR PremixExTaq II (manufactured by Takara Bio Inc.) in accordance with manufacturer's instructions. Primers described below were used. As a result of RT-PCR, both the inflammatory cytokines IL-6 and IL-1b exhibited significantly high expression in the leg on the incision side as compared with the leg on the sham side. Thus, the model rat with tendon injury was confirmed to significantly induce inflammation in the leg on the incision side as compared with the leg on the sham side. Of these inflammatory cytokines, IL-6 which exhibited the most stable results was used as the target of subsequent experiments on the anti-inflammatory effect.

### (Primer set for rat IL-6)

Forward primer (F): TCTCTCCGCAAGAGACTTCCA (SEQ ID NO: 1)
Reverse primer (R): GGTCTGTTGTGGGTGGTATCC (SEQ ID NO: 2)

### (Primer set for rat IL-1b)

Forward primer (F): TGTGATGTTCCCATTAGAC (SEQ ID NO: 3)
Reverse primer (R): AATACCACTTGTTGGCTTA (SEQ ID NO: 4)

In each model rat with tendon injury, the right leg was used as the sham side while the left leg was used as the incision side. After excision was made near an Achilles tendon attachment site, the wound was closed, and 50 µL of PRP containing 1.0 × 10⁷ cells of platelet (PRP administration group; control group) or 50 µL of ASCL-PLC containing 1.0 × 10⁷ cells of platelet (ASCL-PLC administration group) was administered thereto. Both the ASCL-PLC and the PRP were stimulated with calcium by incubation with 10 mM CaCl₂ in PBS (phosphate buffered saline) for 15 minutes before administration.

Three days after administration, each rat was sacrificed, and the expression level of IL-6 was analyzed by RT-PCR in the leg on the sham side and the leg on the incision side. The results are shown in Figure 1.

As is evident from Figure 1, the ASCL-PLC administration group was found to significantly reduce the expression level of IL-6 as compared with the PRP administration group (control group). This indicated that use of the platelet of the present invention significantly suppresses inflammation ascribable to tendinopathy as compared with the case of using PRP. These results demonstrate that the ASCL-PLC used is markedly superior in therapeutic effect on tendinopathy to a conventional method using PRP.

### 6. [Confirmation of in vitro anti-inflammatory effect of ASCL-PLC]

In order to confirm how the anti-inflammatory effect of ASCL-PLC worked as compared with that of PRP, the following in vitro experiment was conducted by focusing on TGF-β also having an anti-inflammatory action.

1 × 10⁸ cells of ASCL-PLC were stimulated with 10 mM CaCl₂ by incubation for 15 minutes in PBS (phosphate buffered saline), and then, an amount (pg) of TGF-β extracellularly released into the PBS by platelet activated by stimulation was measured. On the other hand, as a control, PRP containing 1 × 10⁸ cells of platelet was stimulated with 10 mM CaCl₂ by incubation for 15 minutes in PBS (phosphate buffered saline), and then, an amount (pg) of TGF-β in the PBS was measured with a commercially available ELISA kit. The results of measuring TGF-β are shown in Figure 2.

As is evident from Figure 2, the platelet contained in the PRP did not produce TGF-β by cell stimulation with calcium, whereas the ASCL-PLC was found to produce TGF-β by cell stimulation with calcium. These results demonstrate that the ASCL-PLC used is markedly superior in therapeutic effect on tendinopathy to a conventional method using PRP.

### 7. [Preparation of model rat with tendon degeneration]

In order to confirm mainly the tendon repair effect of the platelet according to the present invention, tendon degeneration models were adopted. Collagenase type 1 (500 UI) having a tendon degenerative effect was administered at 50 µL to each of both the legs of each 8-week-old rat, and the rat after 2 weeks for which degeneration sufficiently progressed was used as a model rat with tendon degeneration.

### 8. [Maximum breaking load test]

In each model rat with tendon degeneration, 50 µL of PBS was administered as a control to the right leg while 50 µL of ASCL-PLC was administered to the left leg. After 2 weeks and after 4 weeks from the administration of ASCL-PLC or PBS, each rat was sacrificed. The Achilles tendon with heel bone was collected from the legs of the sacrificed rat. Both the stumps were held by load cells and placed under traction stress, and strength at break was compared. The results of the maximum breaking load test obtained after 2 weeks from the administration of ASCL-PLC or PBS are shown in the left panel of Figure 3, and the results thereof obtained after 4 weeks are shown in the right panel of Figure 3.

As is evident from Figure 3, the ASCL-PLC administration group improved the maximum strength of the Achilles tendon as compared with the control group, and showed significant improvement after 4 weeks.

### 9. [Histological evaluation]

In each model rat with tendon degeneration, 50 µL of PBS was administered as a control to the right leg while 50 µL of ASCL-PLC was administered to the left leg. After 1 week, 2 weeks, and 4 weeks from administration, each rat was sacrificed. The legs of the sacrificed rat were contrived so as to extend the Achilles tendon, and fixed in formalin. The fixed Achilles tendon was stained with hematoxylin-eosin (HE), Alcian Blue, and Picrosirius Red.

The results of staining the fixed Achilles tendon with HE are shown in Figure 4, and the results of staining the fixed Achilles tenson with Alcian Blue are shown in Figure 5. The results of staining the fixed Achilles tendon with Picrosirius Red and observing it under a polarizing microscope are shown in Figure 6. As a result of HE staining, the ASCL-PLC administration group was confirmed to improve a collagen alignment as compared with the PBS administration group (control group). As a result of Alcian Blue staining, the ASCL-PLC administration group had a low degree of staining and was confirmed to improve tendon degeneration, as compared with the PBS administration group (control group). As a result of Picrosirius Red staining, the ASCL-PLC administration group had more uniform stainability and was confirmed to improve tendon degeneration, as compared with the PBS administration group (control group).

Bonar scores were further calculated from the results of hematoxylin-eosin staining and Alcian Blue staining, and the results about the PBS administration group (control group) and the ASCL-PLC administration group were compared on a weekly basis after administration. The results are shown in Figure 7. The Bonar scores are known as the most general method for pathological evaluation of the tendon (for example, JaworskiL, et al; J Clin Med. 2022 Mar 13; 11 (6); 1572).

As is evident from Figure 7, the ASCL-PLC administration group was confirmed after 4 weeks from administration to significantly decrease the Bonar score and significantly improve tendinopathy histologically, as compared with the PBS administration group (control group).

### 10. [Analysis of expression level of tendon-related cytokine in model rat with tendon degeneration]

An improving effect on tendinopathy was verified by analyzing expression levels of tendon-related cytokines by RT-PCR using model rats with tendon degeneration. Col-1 (collagen type 1), Col-3 (collagen type 3), SCX (scleraxis), and TNC (tenascin C) were adopted as the tendon-related cytokines.

In each model rat with tendon degeneration, 50 µL of PBS was administered as a control to the right leg while 50 µL of ASCL-PLC was administered to the left leg. After 1 week, 2 weeks, and 4 weeks from administration, the model rat with tendon degeneration was sacrificed, and the expression levels of the tendon-related cytokines (i.e., Col-1, Col-3, SCX, and TNC) were analyzed by RT-PCR in the ASCL-PLC administration group (left legs) and the control group (PBS administration group) (right legs). Primers described below were used, and RT-PCR (intercalator method) was performed in the same manner as the method described above in 5. of Examples. The results of RT-PCR are shown in Figure 8.

### (Primer set for rat collagen type 1)

Forward primer (F): GCGTAGCCTACATGGACCAACAG (SEQ ID NO: 5)
Reverse primer (R): AAGTTCCGGTGTGACTCGTGCAG (SEQ ID NO: 6)

### (Primer set for rat collagen type 3)

F: TCGGGATCTGTCCTCTGTGATGA (SEQ ID NO: 7)
R: CAGGAATGACAGGAGCAGGTGTA (SEQ ID NO: 8)

### (Primer set for rat SCX)

F: AGCAACCAGAGAAAGTTGAGCAAAG (SEQ ID NO: 9)
R: TTCCACCTTCACTAGTGGCATC (SEQ ID NO: 10)

### (Primer set for rat TNC)

F: TGATGGAGGTGGATGGATCGTT (SEQ ID NO: 11)
R: CTCAGGTTATCCAGTCCAAGCCA (SEQ ID NO: 12)

As is evident from Figure 8, the ASCL-PLC administration group was confirmed after 4 weeks from administration to significantly improve the expression levels of all of Col-1, Col-3, SCX, and TNC as compared with the PBS administration group (control group). Thus, the ASCL-PLC administration group was confirmed to improve a repair action on tendon degeneration as compared with the PBS administration group (control group).

### 11. [Confirmation of in vitro tendon repair effect of ASCL-PLC]

In order to confirm how the tendon repair effect of ASCL-PLC worked, the following in vitro experiment was conducted using tenoblasts.

Tenoblasts were contacted with PBS (phosphate buffered saline) containing 1 × 10⁸ cells of ASCL-PLC for 3 hours, 6 hours, or 12 hours, followed by the measurement of an amount of Col-1 in the PBS. On the other hand, a test was conducted as a control test in the same manner as above without the use of ASCL-PLC, followed by the measurement of an amount of Col-1. These results are shown in Figure 9.

As is evident from Figure 9, use of the ASCL-PLC significantly improved the expression of Col-1 after 6 hours and 12 hours. This demonstrated that the ASCL-PLC has an effect of promoting the differentiation of tenoblasts.

### Industrial Applicability

The present invention can provide, for example, a therapeutic agent for tendinopathy using platelet produced from a specific mesenchymal stem cell line derived from an adipose tissue (ASCL), the therapeutic agent stably having an excellent therapeutic effect on the tendinopathy. The platelet of the present invention has the advantages that, for example, the platelet can be cultured in a large amount and can also be cryopreserved because the platelet is prepared from ASCL.

## Claims

1. A therapeutic agent for tendinopathy, the therapeutic agent comprising platelet as an active ingredient, the platelet having been produced by a production method comprising the following steps (A) to (C):
(A) inducing one or more cells selected from a stromal vascular fraction of a vertebrate animal adipose tissue comprising a mesenchymal stem cell, an adipose progenitor cell, and a stromal cell to differentiate into a mature adipocyte;
(B) inducing the mature adipocyte obtained in step (A) to dedifferentiate to obtain a mesenchymal stem cell line derived from the vertebrate animal adipose tissue; and
(C) culturing the mesenchymal stem cell line derived from the adipose tissue obtained in step (B) in a medium for inducing differentiation into megakaryocytic cells, and collecting platelet from the cultures, the medium comprising an iron ion and an iron transporter.

2. The therapeutic agent according to claim 1, wherein the step of inducing one or more cells to differentiate into a mature adipocyte in step (A) is a step of culturing the one or more cells in a basal medium for mesenchymal cell culture, the basal medium comprising one or more adipose cell differentiation inducing substances selected from the group consisting of dexamethasone, isobutylmethylxanthine, insulin, and serum.

3. The therapeutic agent according to claim 1, wherein inducing the mature adipocyte to dedifferentiate in step (B) is performing a ceiling culture of the mature adipocyte.

4. The therapeutic agent according to claim 1, wherein the one or more cells are a cell or cells obtained by removing the mature adipocyte from a cell population obtained by treating the vertebrate animal adipose tissue with an enzyme capable of dispersing vertebrate animal adipose tissue cells.

5. The therapeutic agent according to claim 4, wherein the enzyme capable of dispersing the vertebrate animal adipose tissue cells is one or more enzymes selected from the group consisting of collagenase, trypsin, caseinase, clostripain, trypsin-EDTA, dispase, thermolysin, pronase, hyaluronidase, pancreatin, elastase, and papain.

6. The therapeutic agent according to claim 1, wherein the iron transporter in step (C) is transferrin.

7. The therapeutic agent according to claim 1, wherein the iron ion and the iron transporter in step (C) are transferrin bound to iron.

8. The therapeutic agent according to claim 1, wherein the therapeutic agent is administered to the tendon of a vertebrate animal in need of treatment of the tendinopathy.

9. The therapeutic agent according to claim 1, wherein the tendinopathy is selected from the group consisting of tendinosis, tendinitis, and tenosynovitis.

10. The therapeutic agent according to claim 1, wherein the tendinopathy is selected from the group consisting of Achilles tendinopathy, patellar tendinopathy, lateral epicondylitis, medial epicondylitis, plantar fasciitis, and rotator cuff tendinopathy.

11. The therapeutic agent according to any one of claims 1 to 10, wherein the vertebrate animal is a human.
